# EUROPEAN PATENT SPECIFICATION

(11) **EP 2 903 555 B1**
(45) Date of publication and mention of the grant of the patent: **22.12.2021**
(21) Application number: 13770946.5
(22) Date of filing: 02.10.2013
(51) Int. Cl.: A61C 13/00

(54) **MOULD FOR DENTAL RESTORATION**
FORM FÜR ZAHNERSATZ
MOULE POUR UNE RESTAURATION DENTAIRE

(30) Priority: 02.10.2012 EP 12306202; 15.10.2012 DK 201270628; 15.10.2012 US 201261713934 P
(43) Date of publication of application: 12.08.2015
(73) Proprietor: 3Shape A/S, 1060 Copenhagen K (DK); CROWNCERAM, 68310 Wittelsheim (FR)
(72) Inventor: FISKER, Rune, 2830 Virum (DK); RAPP, Frederic, 68120 Pfastatt (FR)
(74) Representative: Guardian IP Consulting I/S
(86) International application number: PCT/EP2013/070552
(87) International publication number: WO 2014/053549

(56) References cited:
- US-A1- 2009 233 258
- US-A1- 2010 119 996

## Description

### Field of the invention

This invention generally relates to a method of manufacturing a dental restoration for a patient.

### Background of the invention

US2004254667A discloses a method for producing a dental restoration wherein a tooth preparation area is scanned either directly or indirectly. The scanned data is used by a computer controlled milling machine to create two or more investment blocks for preparing a suitable dental restoration, the investment blocks being of a material which may be used in ovens operating in a 800 degrees to 1,200 degrees centigrade range. A suitable dental restorative material and the blocks are placed in an oven, and after being clamped together, the material is pressed or injected into the investment blocks to form the desired dental restoration. Subsequently, after cooling, the dental restoration is divested from the investment mold and finished.

WO12068581A discloses that a dental prostheses may be manufactured based on electronic models of the prostheses. Manufacturing aids may be used to check manufacturing quality of the dental prosthesis and components thereof. For example, a jig may be used during and after the manufacture of the dental prostheses and systems and methods for using the same. A customized tooth die may be designed and fabricated to fit a dental prosthesis or components thereof in a jig for inspection. A customized jig may be designed and fabricated for inspecting a dental prosthesis or components thereof throughout the manufacturing process.

US2009/233258 discloses a method of manufacturing a dental mould.

US2004245663A discloses that a ceramic tape is provided in its green state so that it is malleable and formable to a mold for forming a dental restoration, but will not break or crack as it is applied to the mold. Pressure may be applied to further form or adapt the ceramic tape to the shape of the mold. Heat is applied simultaneously with pressure or in a separate step to achieve high density and strength in the ceramic material. A vacuum atmosphere may be used with the application of pressure and/or heat. One or more layers of surface material such as porcelain or composite resin may be applied to the ceramic to form the dental restoration.

It remains a problem to provide an improved method for designing a mould adapted to be used for manufacturing a dental restoration.

It remains a problem to provide a method for designing a virtual mould used for manufacturing a corresponding physical mould adapted to be used in manufacturing of a dental restoration for a patient, where the mould comprises a retention structure which is applicable in retaining a first portion of the dental restoration in the manufactured mould

It remains a problem to provide an improved method for designing a virtual mould used for manufacturing a corresponding physical mould adapted to be used in manufacturing of a dental restoration for a patient

It remains a problem to provide an improved method for manufacturing a mould for a dental restoration comprising two or more layers.

### Summary

Disclosed is a method for designing a virtual mould used for manufacturing a corresponding physical mould adapted to be used in manufacturing of a dental restoration for a patient, wherein the method comprises:
- obtaining a 3D representation of the patient's set of teeth comprising a restoration site where the manufactured dental restoration is adapted to be arranged;
- virtually designing at least a first layer and a second layer of the dental restoration, where the first layer is part of a first portion of the dental restoration, and where the combination of the first portion and the second layer provides at least part of the dental restoration; and
- virtually designing the mould, which is adapted for the manufacturing of the second layer, where the inside surface of the mould is adapted to match the combination of the first portion and the second layer, where the inside surface of the mould corresponds to the shape of the dental restoration;
where virtually designing the mould comprises designing a virtual retention structure of the mould, where the corresponding physical retention structure is applicable in retaining the first portion of the dental restoration in its correct position in the mould.

Disclosed is a method for use in manufacturing a dental restoration for a restoration site of a patient, said dental restoration comprising a first layer and a second layer, the method comprising:
- designing a virtual mould using the method according to an embodiment of the method for designing a virtual mould;
- obtaining a first portion of the dental restoration, said first portion comprising the first layer;
- manufacturing a physical mould from the designed virtual mould, where the physical mould is adapted to be used in manufacturing of the second layer of the dental restoration and comprises a retention structure applicable for retaining the first portion of the dental restoration in its correct position in the mould;
- arranging the first portion of the dental restoration inside the physical mould such that it is held in its correct position by use of said retention structure; and
- manufacturing the second layer of said dental restoration using the manufactured physical mould with the first portion arranged inside.

In embodiments where the dental restoration is a denture, the first portion may be the denture teeth and the second layer may be the gingiva part of the denture adapted to match the restoration site.

In embodiments where the dental restoration is a crown, a bridge, an inlay, an onlay, an implant bridge or an implant crown, the first layer may be adapted to match the restoration site.

The first portion is retained in the mould to prevent that it is displaced or moved when wax, ceramics or acrylic materials are applied in the mould to form the second layer. This provides that the first portion and the second layer are arranged correct relative to each other when the second layer of the dental restoration is formed.

It is an advantage that the shape of the entire final restoration, e.g. a dental restoration consisting of the portion and the second layer, is made in the mould, because then a dental technician does not need to do any manual work on the restoration after the lost-wax process and pressing of ceramics, which is required in prior art.

Furthermore, it is an advantage that the entire restoration can be made digitally and automatically without any manual work, since this will provide a better result with a more accurate restoration, a better fit to the restoration site etc.

In the contexts of the present invention, the phrase "restoration site" refers to a site in the patient's set of teeth where the dental restoration is to be seated. This can e.g. be at a tooth prepared for accepting a crown restoration or at an abutment secured in a dental implant. When the mould is for manufacturing the gingiva part of a denture gingiva the restoration site may comprise the soft tissue where the denture is to be placed.

In some embodiments, the first layer is adapted to match the restoration site, and is thus the layer in the "bottom" of the mould. One side of the first layer is thus adapted to face the restoration site and match it or fit to it when the first portion of the manufactured dental restoration is arranged in its correct position. The other side of the first layer is adapted to face the layer on top of it in the restoration, which can for example be the second layer, if there are only these two layers, or it can be a third layer, if there are more than two layers in the restoration, or it can be an opaque coating which is applied to the first layer. The second layer is then the layer in the "top" of the mould. Thus the second layer is the outermost layer of the restoration, the layer on the tip of the restoration. The second layer is thus arranged opposite to the first layer in the restoration, and the second layer can be termed the coronal layer of the restoration, or the layer in the coronal direction of the restoration. The second layer will at least partly cover or encapsulate the first layer, maybe with a third layer, fourth layer etc. in between. The second layer will typically be wider and broader than the first layer, but the second layer can also be smaller than the first layer, for example if the second layer does not extend down the sides of the restoration, but only is arranged on the top of the restoration.

Hereby the combination of the first layer and the second layer will match the inside surface of the mould, even if there are additional layers, such as a third layer, a fourth layer etc. arranged between the first layer and the second layer. Thus the first layer and the second layer are not necessarily neighbor layers, but may be arranged with one or more layers in-between. Thus the order of the layers may for example be the first layer, the third layer, the fourth layer, and then the second layer.

The additional layers, such as a third and fourth layer, and an opaque coating, may also be part of the first portion of the dental restoration such that the second layer manufactured by the mould is adapted to fit onto the first portion comprising the first layer and the additional layers.

According to an aspect, disclosed is a method for designing a virtual mould used for manufacturing a corresponding physical mould adapted to be used in manufacturing of a dental restoration for a patient, wherein the method comprises:
- obtaining a 3D representation of the patient's set of teeth comprising a restoration site where the manufactured dental restoration is adapted to be arranged;
- virtually designing at least a first layer and a second layer of the dental restoration, where the first layer is adapted to match the restoration site, and where the combination of the first layer and the second layer provides at least part of the dental restoration; and
- virtually designing the mould, which is adapted for the manufacturing of the second layer, where the inside surface of the mould is adapted to match the combination of at least the first layer and the second layer, and where the inside surface of the mould corresponds to the shape of the dental restoration.

According to an aspect, disclosed is a method for designing a virtual mould used for manufacturing a corresponding physical mould adapted to be used in manufacturing a dental restoration for a patient, wherein the method comprises:
- obtaining a 3D representation of the patient's set of teeth comprising a restoration site where the manufactured dental restoration is adapted to be arranged;
- virtually designing a frame layer and an anatomy layer of the dental restoration, where the combination of the frame layer and the anatomy layer provides at least part of the dental restoration; and
- virtually designing a mould, where the inside surface of the mould is adapted to match the combination of the anatomy layer and the frame layer, whereby the inside surface of the mould corresponds to the shape of the dental restoration.

According to an aspect, disclosed are similar methods as above for manufacturing a dental restoration for a patient.

### Designing layers

The innermost surface of the first layer may be designed based on the obtained a 3D representation of the patient's set of teeth, e.g. by copying or offsetting the part of the obtained 3D representation corresponding to the restoration site.

In some embodiments the method comprises virtually designing a third layer of the dental restoration.

The third layer may be comprised in the first portion of the dental restoration, or be a separate layer which is arranged between the first and the second layer.

When dental restoration is designed such that the second and third layers are adjacent, the retention structure of the mould may be a retention pin configured for engaging the outer surface of the third layer.

In some embodiments the method comprises virtually designing a fourth layer of the dental restoration.

The fourth may be comprised in the first portion of the dental restoration.

Furthermore, a fifth layer, a sixth layer etc. of the dental restoration may be virtually designed.

Dividing the dental restoration into more layers than the first and second layers provides a larger degree of freedom in designing the dental restoration to have an appearance which closely matches that of a natural tooth.

In some embodiments the method comprises designing the second layer before designing the first layer.

In this case the second layer can be designed to provide a desired shape of the outermost surface of the dental restoration while taking into account desired mechanical and optical properties of the second layer. For instance a minimum thickness of the second layer is preferred in order to provide a mechanical robust second layer with a certain shade of this layer.

In some embodiments the method comprises designing the first layer before designing the second layer.

In this case the first layer can be designed to provide e.g. a minimum thickness of the first layer in order to provide a mechanical robust first layer configured for engaging the restoration site of the patient's set of teeth.

In some embodiments the method comprises designing the first layer and the second layer, before designing the third layer.

In some embodiments the method comprises designing the third layer before designing the first layer and the second layer.

This order of designing the layer provides an advantage when the third layer has a significant impact on the visual appearance of the dental restoration, such as when the third layer defines the mamelon structure of a crown restoration. For a three layer dental restoration, the second layer can be designed from the outer surface of the third layer and the desired shape of the dental restoration, while the first layer can be designed from the inner surface of the third layer and from the restoration site part of the obtained 3D representation of the patient's set of teeth.

In some embodiments the method comprises designing the first layer and the second layer, before designing the fourth layer.

In some embodiments the method comprises designing the third layer before designing the fourth layer.

In some embodiments the method comprises designing the fourth layer before designing the third layer.

Many combinations of the order of designing the different layers exists, however the ones mentioned above are the most likely combinations, for example designing at least the part of the first layer facing the restoration site first because the restoration site which the first layer shall match is known from the obtained 3D representation of the patient's set of teeth. Furthermore, designing at least the part of the second layer defining the outer surface of the dental restoration at the incisal surface or occlusal surface, and the lingual and buccal surfaces of the restoration before designing any third layer or fourth layer is also a likely order, since the incisal or occlusal surface, and the lingual and buccal surfaces of the restoration, should typically be determined before it makes sense to design any layers underneath the second layer.

### Kinds of layers

In some embodiments the first layer is a frame layer, such as a frame layer for a removable partial denture.

If there should be a frame in the restoration, the frame layer will typically be the first layer. However, a frame layer is not required in all restorations, so the first layer may also be a dentin or mamelon layer.

In some embodiments the first layer is a coping, such as the coping of a single restoration crown or the coping of a crown portion of a bridge.

In some embodiments one of the layers is an anatomy layer, an incisal layer or an occlusal layer of the dental restoration.

This can for example be the second layer or the third layer or the fourth layer.

In some embodiments one of the layers is a dentin or mamelon layer.

This can for example be the third layer if there is a frame layer in the restoration. If there is no frame layer in the restoration, the dentin or mamelon layer may be the first layer.

It is advantageous that one of the layers in the dental restoration is a dentin or mamelon layer since this improves the visual appearance of the restoration which becomes more lifelike when a layer mimics the shape of the patient's natural dentin layer or mamelon layer.

A mamelon layer can be designed by selecting a template from a mamelon structure library and modifying the template to the patient, e.g. by increasing the height of the indentations in the mamelon structure.

In some embodiments one of the layers is an incisal layer.

This can for example be the second layer.

In some embodiments one of the layers is a deep dentin layer.

This can for example be the third layer.

In some embodiments one of the layers is a higher dentin layer.

This can for example be the fourth layer. The second layer may then be an incisal layer, which can be the top layer of a restoration.

In some embodiments, the first layer is the teeth of a denture and the second layer comprises the gingiva part of the denture.

In embodiments where the dental restoration consists of the first layer and the second layer, the first portion of the dental restoration consists of the first layer. In such cases, the comments relating to the first layer may also be valid for the first portion and vice versa.

The first portion is not necessarily a coherent structure. For example in cases where the dental restoration is a removable partial denture, the mould may be for the manufacture of the gingiva layer of the denture and the first portion may comprise the denture teeth and the framework of the removable partial denture, such as the retention grid, the major and minor connectors, and the clamps if the removable partial denture.

### Manufacturing of layers

In some embodiments the first layer is manufactured by milling or casting. For some dental restorations, the first layer is typically made of metal, and is manufactured by means of milling or casting.

In some embodiments the first layer is manufactured in a first mould.

If the first layer is not a frame layer, then the first layer may be manufactured in its own mould, denoted the first mould, before it is arranged in the mould for the manufacturing of the second layer.

In some embodiments the third layer is manufactured in a third mould.

Thus the third layer may be manufactured in its own mould, denoted the third mould, before it is arranged in the mould for the manufacturing of the second layer.

In some embodiments the fourth layer is manufactured in a fourth mould. Thus the fourth layer may be manufactured in its own mould, denoted the fourth mould, before it is arranged in the mould for the manufacturing of the second layer.

### Manufacturing

In some embodiments the first portion of the dental restoration comprising the first layer is adapted to be arranged in the mould for manufacturing of the second layer, before the second layer is manufactured by means of the mould.

Since the mould is designed to match at least the first layer and the second layer, the first layer fits in the mould, since the mould is shaped to accommodate the first layer. When the first layer is arranged in the mould, then the second layer can be manufactured, either as a wax copy of the second layer or as the actual second layer of e.g. ceramics or acrylic materials. If a third layer, fourth layer etc. should also be part of the restoration, then these layers may also be arranged in the mould for manufacturing of the second layer, before the second layer is manufactured. A second layer of ceramics or acrylic materials may chemically bond to the layer underneath, which may be the first layer or the third layer. When the first portion comprises both the first layer and a third layer these layers have typically been chemically bonded to define the first portion of the dental restoration before the second layer is manufactured.

In some embodiments the method comprises arranging the first portion of the dental restoration in the mould for manufacturing of the second layer, before the second layer is manufactured by means of the mould.

In particular, disclosed herein is a method for use in manufacturing a physical mould adapted to be used in manufacturing of a dental restoration for a patient, wherein the method comprises:
- designing a virtual mould using the method according to an embodiment of the method for virtually designing a mould, and
- manufacturing the physical mould based on the designed virtual mould.

Disclosed is a method for manufacturing of a dental restoration for a patient from a physical mould, wherein the dental restoration comprises a first portion and a second layer, the method comprising:
- designing a virtual mould used for manufacturing the corresponding physical mould, wherein the virtual mould is designed using a method according to an embodiment of the invention;
- manufacturing the physical mould based on the virtual mould;
- obtaining a first portion of the dental restoration;
- arranging said first portion inside the manufactured mould; and
- providing the material of the second layer into the mould such that the second layer is shaped by the inside surface of the mould and the surfaces of the first portion.

In some embodiments the method further comprises:
- manufacturing at least the first layer;
- manufacturing the mould as a mould for wax injection;
- arranging at least the first layer in the mould;
- injecting wax into the mould;
- allowing the wax to harden to the shape of the second layer in the mould; and
- removing the mould around the hardened wax.

In some embodiments, the physical mould is manufactured as a mould for wax injection, and the method comprises
- injecting wax into the mould;
- allowing the wax to harden to the shape of the second layer in the mould; and
- removing the mould around the hardened wax.

This provides that a wax copy of the second layer is provided which can be used in subsequent steps of the manufacture of the dental restoration.

In some embodiments the method further comprises:
- manufacturing at least the first layer;
- manufacturing the mould as an mould for ceramic pressing, such as an investment mould or a metal mould;
- arranging at least the first layer in the mould;
- pressing ceramics into the mould;
- allowing the ceramics to cool and harden to the shape of the second layer in the mould; and
- removing the mould around the hardened ceramics.

In some embodiments, the physical mould is manufactured as a mould for ceramic pressing, such as an investment mould or a metal mould, and the method comprises:
- pressing ceramics into the mould;
- allowing the ceramics to cool and harden to the shape of the second layer in the mould; and
- removing the mould around the hardened ceramics.

It is an advantage that the first layer is placed in the mould and that wax, ceramics or an acrylic material is filled into the mould, because hereby the wax, the ceramics or the acrylic material will acquire the entire shape of the second layer of the restoration, whereby a dental technician does not have to manually finish areas of the restoration, as in prior art.

The mould is for the manufacturing of the second layer. If the restoration should comprise more layers than just the first and second layer, then the third layer, fourth layer etc. are also manufactured, so that all these layers, e.g. the first layer, the third layer and the fourth layer, are arranged in the mould, either as separate layers or as the first portion of the dental restoration when this comprises these layers.

In some embodiments the method further comprises:
- manufacturing the frame layer;
- manufacturing the mould;
- arranging the frame layer in the mould;
- injecting wax into the mould;
- allowing the wax to harden to the shape of the anatomy layer in the mould; and
- removing the mould around the hardened wax.

It is an advantage that the frame layer is placed in the mould and that wax is filled into the mould, because hereby the wax will acquire the entire shape of the anatomy layer of the restoration, whereby a dental technician does not have to manually finish areas of the restoration, as in prior art.

In some embodiments arranging the first portion in the mould comprises arranging the first portion such that the first layer is in its correct position in the mould, where the first layer matches the shape of the mould.

In some embodiments the first layer is manufactured in a first mould.

In some embodiments arranging the frame layer in the mould comprises arranging the frame layer in its correct position in the mould, where the frame layer matches the shape of the mould.

### Overpressing

In some embodiments the method further comprises:
- arranging the hardened wax in a container suitable for resisting high pressure and high temperature, where the container comprises an inlet for gypsum, an outlet for wax, and an inlet for ceramics;
- providing gypsum into the container;
- melting out the wax of the container after the gypsum has hardened;
- pressing in ceramics in the container, where the ceramics will acquire the shape of the lost wax; and
- removing the container and the gypsum around the ceramics, whereby the ceramics will have obtained the shape of the designed second layer of the dental restoration.

It is an advantage that due to the lost-wax process, the ceramics will acquire the exact shape of the lost wax, and since the lost wax had the entire shape of the final second layer because the wax was originally shaped in the mould, the ceramics will acquire the final shape of the second layer of the restoration. Thus a dental technician does not have to manually finish areas on the second layer, in particular the areas at the margin line, which the conventional processes cannot automatically cover.

In some embodiments the method further comprises:
- arranging the hardened wax in a container suitable for resisting high pressure and high temperature, where the container comprises an inlet for gypsum, an outlet for wax, and an inlet for ceramics;
- providing gypsum into the container;
- melting out the wax of the container after the gypsum has hardened;
- pressing in ceramics in the container, where the ceramics will acquire the shape of the lost wax; and
- removing the container and the gypsum around the ceramics, whereby the ceramics will have obtained the shape of the designed anatomy layer of the dental restoration.

### Sprues

In some embodiments the method further comprises virtually designing sprues for the outlet of melted wax and/or for the inlet of ceramics or acrylic materials.

A sprue is the passage through which the liquid ceramics or acrylic materials is introduced into the container or the investment mould. During the pressing or moulding, the liquid in the sprue will solidify and need to be removed from the finished restoration e.g. after the ceramic pressing. A sprue may also be the passage through which the melted wax exits the container. In the container the same sprue or sprues may be used as both the wax outlet and the ceramic/acrylic inlet.

The sprues may be designed on the mould for wax injection, and the sprues may be designed inside the investment mould for ceramic pressing.

In particular, it may be an advantage to digitally design the sprues for a large restoration, such as a full arch bridge etc., because here several sprues may be needed, and the material and time which can be saved due to the digital design of the sprues may thus be considerable.

It is an advantage to virtually design the sprues, because hereby the length of the sprues can be reduced, which is an advantage since material can be saved, and if the sprues are printed in wax, then a considerable amount of time can also be saved when only a short sprue should be printed instead of a longer sprue, since the time it takes to print a sprue is about linearly or directly proportional to the length of the printed sprue.

In some embodiments the sprues are printed in wax.

If a wax copy of the restoration also should be provided, the sprues and the wax copy may both be printed in wax. Alternatively, the restoration may be moulded directly in ceramic without making a wax copy, and in this case just the sprues may be printed in wax.

Traditionally, sprues have been attached to the wax copy of the restoration by heating up the end of the sprue and then melting the end onto the wax copy. When the wax is melted the exact and original shape of the surface may not be maintained or it may be difficult to exactly determine the original surface. When the restoration of ceramics have been manufactured, the ceramic sprue should now be cut of the surface of the restoration, but since the exact and original shape of the surface was not maintained in the wax step or since it is difficult to determine the original surface, it may be difficult to cut the sprue off correctly, and the surface of the restoration may therefore be partly destroyed by the sprue.

It is therefore an advantage to solve this problem.

In some embodiments the method comprises virtually designing a protrusion inside the mould. If the mould is used for forming a wax copy of the second layer by wax injection, the protrusion can define a hole in the wax copy.

When a hole is formed in the wax copy, then no sprue should be melted onto the surface of the wax copy, and the surface of the wax copy is therefore not destroyed. The sprue can be arranged in the hole instead.

In some embodiments the method comprises virtually designing an opening in the mould. If the mould is used for forming a wax copy of the second layer by wax injection, a pin can be arranged in the opening such that the pin defines a hole the wax copy of the second layer.

When a hole is formed in the wax copy, then no sprue should is melted onto the surface of the wax copy, and the surface of the wax copy is therefore not destroyed. The sprue can be arranged in the hole instead.

In some embodiments the method comprises virtually designing a sprue on the outside surface of the mould and the mould is for wax injection.

When the sprue is designed as part of the surface of the mould, then no sprue should melted onto the surface of the wax copy, and the surface of the wax copy is therefore not destroyed.

### Retention structure for first portion inside mould

The method comprises providing a retention means, such as a retention structure, applicable for retaining the first portion of the dental restoration in the mould.

The first portion is retained in the mould, such that the first portion is not displaced or moved when wax, ceramics, or acrylic materials are applied in the mould. It is important that the first layer and the following layer, e.g. the third or second layer, are arranged correct relative to each other.

In some embodiments, the virtual retention structure comprises a virtual vacuum suction hole arranged at the part of the virtual mould corresponding to the part of the physical mould where the first layer is to be arranged. The virtually designing then comprises defining said vacuum suction hole in the virtual mould e.g. by a Boolean subtraction of an appropriate virtual structure. Vacuum suction can then be applied through the vacuum suction hole of the manufactured mould to ensure that the first portion of the dental restoration is retained in its correct position during the manufacture of the second layer

In some embodiments the retention means is a vacuum suction arranged such that the vacuum suction is adapted to provide suctions through a hole in the mould. In some embodiments the retention structure comprises a vacuum suction hole and the method of manufacturing the dental restoration comprises providing vacuum suction through the vacuum suction hole such that the first portion of the dental restoration is retained in the correct position in the mould.

The suction should preferably be arranged such that it provides suction on the first layer of the first portion arranged in the mould, such as below the first layer.

In some embodiments the retention means is an adhesive material, such as glue, tape, moist etc.

In some embodiments the method comprises virtually designing a retention structure inside the mould for retaining the first portion in the manufactured mould.

It is an advantage that the first portion can be retained in the mould, such that the first portion is not displaced or moved when wax, ceramics or acrylic materials are applied in the mould. It is important that the first layer and the following layer, e.g. the third or second layer, are arranged correct relative to each other.

In some embodiments the virtual retention structure comprises a virtual retention pin configured to provide that the corresponding physical retention pin of the manufactured mould extends from a point on the inside surface of the mould to a point on the opposing surface of the first portion of the dental restoration, i.e. extending to the opposing surface of the first portion when this is arranged in the mould. The retention pin is then applicable for holding the first portion of the dental restoration in its correct position while the mould is used for manufacture of the second layer.

In some embodiments the retention structure of the manufactured mould comprises a physical retention pin and the first portion is arranged in the mould such that the retention pin engages a surface of the first portion to hold it in the correct position.

The retention pin should be configured for securing and holding on to the first layer in the mould, so the pin may for example be arranged on the opposite part of the mould from where the first layer is arranged in the mould. The mould can be disassembled in for example two parts at the join line at the equator of the mould. The retention pin is configured for engaging the first layer or the third layer if any, or the fourth layer if any, or the opaque coating if any, whichever layer or coating is closest to the second layer in the dental restoration such that the retention pin retains the first layer and the third layer if any, the fourth layer if any, and the opaque coating if any, in the correct position during the manufacture of the second layer.

In some embodiments, the designing of the virtual retention pin is configured to provide that it is an integrated part of the virtual mould, such that the physical retention pin is an integrated part of the physical mould formed when the mould is manufactured.

This has the advantage that the retention pin automatically is arranged correctly in the mould without the need for a skillful operator to arrange the first portion and the retention structure in the manufactured mould.

In some embodiments, virtual designing comprises defining a point on the inside surface of the virtual mould from which the virtual retention pin extends and a point on the opposing surface of a virtual first portion corresponding to the first portion of the dental restoration, and virtually generating the retention pin by forming a structure which extends between these two points.

The point on the inside surface of the virtual mould may be defined by indicating a point on the corresponding surface of the virtually designed second layer since this will coincide with the inner surface of the virtual mould over a large part of the inner mould surface

In some embodiments, virtual designing the retention pin comprises:
- generating a virtual pin structure which intersects the inside surface of the virtual mould and a virtual first portion corresponding to the first portion of the dental restoration; and
- forming the virtual retention pin by subtracting the virtual first portion and the virtual mould from the virtual pin structure.

The subtraction of the virtual first portion and the virtual mould from the virtual pin structure provides that the parts of the virtual pin structure extending into the virtual first portion and beyond the inside surface of the virtual mould are cut away. The formed virtual retention pin is then shaped at its ends according to the virtual first portion and at the inner surface of the virtual mould and extends between the outer surface of the first portion of the dental restoration and the inner surface of the mould such that the physical retention pin can engage and hold the first portion in its correct place in the mould.

In regions where the surface of the virtually designed second layer coincides with the inner surface of the virtual mould, the virtual pin structure may be cut by the outer surface of the second layer instead of the virtual mould. This will give the same result as virtually subtracting the virtual mould from the virtual pin structure.

### Combined surface of second layer and first layer

In some embodiments the method comprises digitally combining the second layer and the first portion or the first layer to a combined surface.

In some embodiments the method comprises digitally combining the first layer, the third layer, and the second layer to a combined surface.

In some embodiments the method comprises digitally combining the first layer, the third layer, the fourth and the second layer to a combined surface.

In some embodiments the method comprises digitally combining the first layer, the third layer if any, the fourth if any and the second layer to a combined surface.

In some embodiments the combined surface comprises the outer surface of the second layer and the jaw facing surface of the first layer.

In some embodiments designing the mould comprises shelling the combined surface.

In some embodiments shelling the combined surface comprises providing an offset of the combined surface outwards its normal vector.

In some embodiments the offset is constant along the combined surface.

In some embodiments the offset varies along the combined surface.

In some embodiments the combined surface is composed of a triangle mesh.

In some embodiments the jaw facing surface of the first layer matches the restoration site of the patient's set of teeth.

In some embodiments the method further comprises digitally combining the anatomy layer and the frame layer to a combined surface.

In some embodiments the combined surface comprises the outer surface of the anatomy layer and the jaw facing surface of the frame layer.

In some embodiments the jaw facing surface of the frame layer matches the restoration site of the patient's set of teeth.

### Opaque coating

In some embodiments the method comprises virtually designing and applying an opaque coating to the first layer on the outer surface of the first layer.

In some embodiments the method comprises virtually designing an opaque coating configured for being applied on the outer surface of the first layer, and where the first portion of the dental restoration comprises said opaque coating.

An opaque coating is applied to the first layer for avoiding that if the first layer is a metal layer, e.g. a frame layer or a coping, the metal color of the first layer can be seen through a partly transparent second layer, third layer, fourth layer etc. Furthermore, the opaque coating provides a chemical bonding between the metal of the first layer and the porcelain or ceramics of the layer above. The layer above may be the second layer if there are only two layers, or the third layer if there are more than two layers.

If the first layer is made of zirconia, zircon, zirconium, zirconium dioxide etc. the an opaque coating also called a liner coating is applied to this first layer for providing a chemical bonding between the zirconia, zircon, zirconium, zirconium dioxide etc. and the layer above, which is typically a porcelain layer, e.g. a veneering. In this case, the opaque coating or liner coating is not for shading the color of the zirconia, zircon, zirconium, zirconium dioxide etc., because the color of zirconia, zircon, zirconium, zirconium dioxide etc. is already tooth-colored.

It is an advantage that the opaque coating or liner coating is designed digitally because in prior art the opaque coating is made manually, whereby it is very difficult to control the thickness of the coating, resulting in that the second layer, third layer, or fourth layer etc. does not fit exactly on the opaque coating on the first layer.

### Mould

In some embodiments the mould is a mould for wax injection.

Thus the mould may be a wax injection mould, where a wax copy of the restoration or of the second layer is manufactured, and after arranging the wax copy in a container, filling in e.g. gypsum, melting out the wax, and filling in ceramics, the final restoration is manufactured.

In some embodiments the mould is an investment mould for ceramic pressing.

Thus the mould may be an investment mould for direct manufacturing of the ceramic restoration, i.e. ceramic is pressed in the investment mould, and the restoration is thus manufactured in a single step, without making a wax copy first.

In some embodiments the mould is a mould for acrylic materials.

The acrylic material can be flexible such that the mould can be used for the manufacture of flexible restorations. This may be advantageous e.g. when the mould is for use in the manufacture of the gingiva part of dentures, such as the gingiva part of full or partial, fixed or removable dentures. For dentures it is often advantageous to manufacture at least the gingiva part of the denture in a flexible acrylic material such that the denture does not cause the patient much discomfort.

For some dental restorations it is advantageous to manufacture the dental restoration in both of metal and an acrylic material. This is e.g. the case for removable partial dentures where parts of the framework, such as the retention grid and the clamps used for securing the denture at the patient's exiting teeth, often are made in a metal such as a chrome cobalt alloy or titanium, and the outer gingiva part and the gingiva part which faces the patient's gingiva can be made in an acrylic material. For example a metal/acrylic denture can be manufactured to be strong, thin, and light such that is cause little discomfort to the patient. Other parts of the removable partial denture

In some embodiments, the first portion comprises denture parts made in metal while the mould is for the manufacture of the denture parts made in acrylics materials.

In some embodiments the mould comprises an assembly structure.

In some embodiments the method comprises designing join lines on the assembly structure of the mould for disassembling the mould for removing the restoration.

This is the case when the mould is an investment mould.

In some embodiments the method comprises designing join lines on the assembly structure of the mould for disassembling the mould for removing the hardened wax or acrylic materials.

This is the case when the mould is a mould for wax injection or for forming the second layer in acrylic materials.

In some embodiments the join lines are arranged on the equator line of the mould.

It is an advantage to arrange the join lines on the equator line of the mould, which is the thickest part of the mould, for ensuring that the restoration or the hardened wax can be removed from the mould. If the mould is not split on the equator line, there is a risk that the restoration or the hardened wax cannot come out of the mould without having to break the mould.

In some embodiments the method comprises virtually designing one or more closing pins at the join lines on the assembly structure.

By means of the closing pins on the assembly structure the mould can be opened and closed without having to break the mould. Avoiding breaking the mould it an advantage because this reduces the risk that the restoration or wax is broken when removed from the mould.

In some embodiments the mould is manufactured in a flexible and/or bendable material.

The flexible and/or bendable material can for example be acrylic, silicone etc. It is an advantage to manufacture the mould in a flexible and/or bendable material, because hereby the wax or the restoration can easily be removed from the mould after manufacture by twisting the mould until the restoration or wax is outside the mould. This can be particularly advantageous for a big restoration such as a full arch bridge etc. where the wax or the restoration is big.

In the context of the present invention, the phrase "a flexible and/or bendable material" refers to a material which can be bended or twisted by a dental technician without the use of excessive force or tools.

### Material for mould

In some embodiments the mould is made of acrylic.

This may the case when the mould is for wax injection.

In some embodiments the mould is made of investment material.

This may be the case when the mould is an investment mould for ceramic pressing.

In some embodiments the mould is made of gypsum.

This may be the case when the mould is an investment mould for ceramic pressing or when the mould is for manufacturing a second layer made of an acrylic material.

When the mould is an investment mould for ceramic pressing, the mould is resistant to high temperatures, such as temperatures up to 1000 degrees Celsius, since a typical temperature for ceramic pressing is 980 degrees Celsius. Furthermore, the investment mould is resistant to high pressure, such as 400 N, since this pressure may typically be applied in ceramic pressing.

In some embodiments the mould is manufactured by rapid manufacturing, such as by 3D printing, milling etc.

Both an investment mould and a mould for wax injection can be printed or milled. The milling or printing should preferably be a process which can be performed relatively fast, so that the step of making the mould is not a time-consuming step in the overall process of manufacturing the restoration.

In some embodiments the mould is made of a metal.

This may e.g. be the case when the mould is for wax injection or when the mould is for manufacturing a second layer made of an acrylic material.

### Entry hole

In some embodiments, the method comprises defining an entry hole in the virtual mould.

The entry hole may be located at the position of the first portion of the dental restoration in the mould.

In some embodiments, the cross sectional area of the entry hole is in the range of about 50 % to about 90 % of the cross sectional area of the first portion of the dental restoration.

Manufacturing the mould in a flexible material and defining the entry hole provides that the first portion of the dental restoration can be pushed into the manufactured mould through said entry hole. Inside the mould, the first portion is supported by the mould material at the edge of the entry hole.

In some embodiments, the mould is manufactured with said entry hole and the first portion of the dental restoration is pushed into the inside of the physical mould through the entry hole.

It is advantageous that the mould is designed and manufactured with an entry hole since the first portion of the dental restoration can be pushed into the manufactured mould through said entry hole. The first portion can then be arranged in a mould which has no split-lines defined to allow the mould to be split into separate parts for arranging the first portion.

In some embodiments, the method comprises virtually designing indentations in the perimeter of the entry hole.

This has the advantage that less pressure must be applied in order to push the first portion into the manufactured mould through said entry hole.

### Dental restoration

In some embodiments the dental restoration is a crown, bridge, inlay, onlay, implant bridge, implant crown, etc.

### Dentures

In some embodiments, the dental restoration is a denture and the first portion comprises the denture teeth while the second layer comprises the gingiva part of the denture. The second layer then shapes the outer surface of the gingiva part of the denture.

In some embodiments, the mould is used for forming a second layer made of acrylic materials such that the gingiva part of the final denture can be manufactured directly from the mould.

This has the advantage the several steps are omitted in the production of the denture which at the same time can be manufactured with a high precision.

In some embodiments, the mould is used for forming a second layer made of wax, such that the gingiva part of a wax try-in denture can be manufactured directly from the mould.

In some embodiments, the mould is used for forming a second layer made of wax and the gingiva part of the final denture can be manufactured in acrylic materials using the lost wax technique based on the second layer of wax.

In some embodiments, the dental restoration is a removable partial denture and the first portion of the dental restoration comprises the denture teeth and the framework of the removable partial denture. The framework may comprise the retention grid, major and minor connectors, and the clamps of the removable partial denture. The framework can at least partially be adapted to match the restoration site, such that e.g. the retention grid is aligned with the soft tissue at the alveolar ridge. Preferably, the framework part of the first portion and the second layer are designed such that the second layer encloses at least the retention grid of the framework. In the manufactured removable partial denture, the retention grid is then integrated in the gingiva part of the denture such that unpleasant contact between the patient's soft tissue and the retention grid is avoided.

In some embodiments, the dental restoration is an implant bar supported denture and the first portion of the dental restoration comprises the denture teeth and the implant bar.

The implant bar is adapted to match the restoration site and to support the gingiva part and teeth of the denture. Preferably, the implant bar and the second layer are designed such that the second layer partly encloses the implant bar such that this is not visible when the denture is secured in the patient's mouth. The implant bar is often produced in a metal such as a chrome cobalt alloy or titanium.

When using the mould for directly manufacturing a denture, the first portion comprising the denture teeth is arranged in the mould and the acrylic material forming the gingival part of the denture is injected into the mould. When the denture is a removable partial denture, the denture framework is part of the first portion and is also arranged in the mould together with the denture teeth when the acrylic material of the denture gingiva is injected into the mould. The same is the case for an implant bar supported denture, where the implant bar is part of the first portion of the dental restoration.

### First portion extending outside mould

In some embodiments, the method comprises virtually removing a part of the first portion and using the remaining part of the first portion when virtually designing the mould.

This can be advantageous in cases where a part of the first portion extends some distance away from where the second layer is to be formed. This may e.g. be the case for a removable partial denture where the clamp parts of the framework which engage the existing teeth in the patient's mouth extend some distance away from the gingiva part of the removable partial denture. Designing the mould to provide that the clamp parts are enclosed in the mould hence makes it unnecessarily complicated and it is hence advantageous that the manufactured mould is shaped such that the clamp parts of the framework are allowed to extend outside the mould.

In some embodiments, virtually removing a part of the first portion comprises virtually dividing the first portion into the part which is to be virtually removed and the remaining part, and the virtual designing the mould comprises forming a virtual opening where the first portion is divided.

This has the advantage that when the physical first portion is arranged in the manufactured mould, the opening allows the part of the physical first portion corresponding to the virtually removed part to extend outside the mould. This may e.g. be a part of a framework clamp.

### ID tags on mould

In some embodiments the method further comprises virtually designing ID tags on the outside surface of the mould.

It is an advantage to provide ID tags on the mould, so that different moulds for different restorations for different patients or clients or clinics, for example, can be kept track off, when more moulds are handled at the same time.

### Support elements on outer surface of mould

In some cases support elements are provided on the virtual mould to ensure that during a 3D printing of the mould this will not bend due to gravitational force pulling down parts of the mould. Such support elements are preferably defined on the outer surface of the mould such that they do not deform the outer surface of the dental restoration.

### Computer implemented method

In some embodiments the method or at least part of the method is computer-implemented.

### 3D scan

In some embodiments the 3D representation is obtained by means of obtaining a 3D scan.

In some embodiments the 3D scan is an intra oral scan of at least part of the patient's set of teeth, a scan of at least part of an impression of the patient's set of teeth, and/or a scan of at least part of a physical model of the patient's set of teeth.

In some embodiments the 3D scan is performed by means of laser light scanning, LED scanning, white light scanning, fluorescence scanning, probe-scanning, X-ray scanning, and/or CT scanning.

### 3D modeling

3D modeling is the process of developing a mathematical, wireframe representation of any three-dimensional object, called a 3D model, via specialized software. Models may be created automatically, e.g. 3D models may be created using multiple approaches: use of NURBS curves to generate accurate and smooth surface patches, polygonal mesh modeling which is a manipulation of faceted geometry, or polygonal mesh subdivision which is advanced tessellation of polygons, resulting in smooth surfaces similar to NURBS models.

### Focus scanning

The intra-oral scanner may be configured for utilizing focus scanning, where the digital 3D representation of the scanned teeth is reconstructed from in-focus images acquired at different focus depths. The focus scanning technique can be performed by generating a probe light and transmitting this probe light towards the set of teeth such that at least a part of the set of teeth is illuminated. Light returning from the set of teeth is transmitted towards a camera and imaged onto an image sensor in the camera by means of an optical system, where the image sensor/camera comprises an array of sensor elements. The position of the focus plane on/relative to the set of teeth is varied by means of focusing optics while images are obtained from/by means of said array of sensor elements. Based on the images, the in-focus position(s) of each of a plurality of the sensor elements or each of a plurality of groups of the sensor elements may be determined for a sequence of focus plane positions.

The in-focus position can e.g. be calculated by determining the light oscillation amplitude for each of a plurality of the sensor elements or each of a plurality of groups of the sensor elements for a range of focus planes. From the in-focus positions, the digital 3D representation of the set of teeth can be derived.

### Desktop scanning

Obtaining a three dimensional representation of the surface of an object by scanning the object in a 3D scanner can be denoted 3D modeling, which is the process of developing a mathematical representation of the three-dimensional surface of the object via specialized software. The product is called a 3D model. A 3D model represents the 3D object using a collection of points in 3D space, connected by various geometric entities such as triangles, lines, curved surfaces, etc. The purpose of a 3D scanner is usually to create a point cloud of geometric samples on the surface of the object.

3D scanners collect distance information about surfaces within its field of view. The "picture" produced by a 3D scanner describes the distance to a surface at each point in the picture.

For most situations, a single a scan or sub-scan will not produce a complete model of the object. Multiple sub-scans, such as 5, 10, 12, 15, 20, 30, 40, 50, 60, 70, 80, 90 or in some cases even hundreds, from many different directions may be required to obtain information about all sides of the object. These sub-scans are brought in a common reference system, a process that may be called alignment or registration, and then merged to create a complete model.

A triangulation 3D laser scanner uses laser light to probe the environment or object. A triangulation laser shines a laser on the object and exploits a camera to look for the location of the laser dot. Depending on how far away the laser strikes a surface, the laser dot appears at different places in the camera's field of view. This technique is called triangulation because the laser dot, the camera and the laser emitter form a triangle. A laser stripe, instead of a single laser dot, may be used and is then swept across the object to speed up the acquisition process.

Structured-light 3D scanners project a pattern of light on the object and look at the deformation of the pattern on the object. The pattern may be one dimensional or two dimensional. An example of a one dimensional pattern is a line. The line is projected onto the object using e.g. an LCD projector or a sweeping laser. A camera, offset slightly from the pattern projector, looks at the shape of the line and uses a technique similar to triangulation to calculate the distance of every point on the line. In the case of a single-line pattern, the line is swept across the field of view to gather distance information one strip at a time.

An example of a two-dimensional pattern is a grid or a line stripe pattern. A camera is used to look at the deformation of the pattern, and an algorithm is used to calculate the distance at each point in the pattern. Algorithms for multistripe laser triangulation may be used.

Iterative Closest Point (ICP) is an algorithm employed to minimize the difference between two clouds of points. ICP can be used to reconstruct 2D or 3D surfaces from different scans or sub-scans. The algorithm is conceptually simple and is commonly used in real-time. It iteratively revises the transformation, i.e. translation and rotation, needed to minimize the distance between the points of two raw scans or sub-scans. The inputs are: points from two raw scans or sub-scans, initial estimation of the transformation, criteria for stopping the iteration. The output is: refined transformation. Essentially the algorithm steps are:
1. Associate points by the nearest neighbor criteria.
2. Estimate transformation parameters using a mean square cost function.
3. Transform the points using the estimated parameters.
4. Iterate, i.e. re-associate the points and so on.

The present invention relates to different aspects including the method described above and in the following, and corresponding methods, devices, apparatuses, systems, uses, kits and/or product means, each yielding one or more of the benefits and advantages described in connection with the first mentioned aspect, and each having one or more embodiments corresponding to the embodiments described in connection with the first mentioned aspect and/or disclosed in the appended claims.

In particular, disclosed herein is a system for designing a virtual mould used for manufacturing a corresponding physical mould adapted to be used in manufacturing of a dental restoration for a patient, wherein the system comprises:
- device for obtaining a 3D representation of the patient's set of teeth comprising a restoration site where the manufactured dental restoration is adapted to be arranged;
- device for virtually designing at least a first layer and a second layer of the dental restoration, where the first layer is adapted to match the restoration site, and where the combination of the first layer and the second layer provides at least part of the dental restoration; and
- device for virtually designing the mould, which is adapted for the manufacturing of the second layer, where the inside surface of the mould is adapted to match the combination of at least the first layer and the second layer, and where the inside surface of the mould corresponds to the shape of the dental restoration.

In particular is disclosed a system for designing a virtual mould used for manufacturing a corresponding physical mould adapted to be used in manufacturing of a dental restoration for a patient, wherein the system comprises:
- device for obtaining a 3D representation of the patient's set of teeth comprising a restoration site where the manufactured dental restoration is adapted to be arranged;
- device for virtually designing at least a first layer and a second layer of the dental restoration, where the first layer is part of a first portion of the dental restoration, and where the combination of the first portion and the second layer provides at least part of the dental restoration; and
- device for virtually designing the mould, which is adapted for the manufacturing of the second layer, where the inside surface of the mould is adapted to match the combination of the first portion and the second layer, where the inside surface of the mould corresponds to the shape of the dental restoration, and where virtually designing the mould comprises designing a virtual retention structure of the mould, where the corresponding physical retention structure is applicable in retaining the first portion of the dental restoration in its correct position in the mould.

In particular, disclosed herein is a system for virtually designing a mould adapted to be used for manufacturing a dental restoration for a patient, wherein the system comprises:
- device for obtaining a 3D representation of the patient's set of teeth comprising a restoration site where the manufactured dental restoration is adapted to be arranged;
- device for virtually designing a frame layer and an anatomy layer of the dental restoration, where the combination of the frame layer and the anatomy layer provides at least part of the dental restoration; and
- device for virtually designing a mould, where the inside surface of the mould is adapted to match the combination of the anatomy layer and the frame layer, whereby the inside surface of the mould corresponds to the shape of the dental restoration.

Furthermore, the invention relates to a computer program product comprising program code means for causing a data processing system to perform the method according to any of the embodiments, when said program code means are executed on the data processing system, and a computer program product, comprising a computer-readable medium having stored there on the program code means.

### Brief description of the drawings

The above and/or additional objects, features and advantages of the present invention, will be further elucidated by the following illustrative and nonlimiting detailed description of embodiments of the present invention, with reference to the appended drawings, wherein:
Fig. 1 shows an example of a flowchart of a method of designing a virtual mould.
Fig. 2 shows an example of a cross-section view of a dental restoration comprising a first layer, a second layer and an opaque coating.
Fig. 3 shows an example of a cross section view of a mould for forming a dental restoration.
Fig. 4 shows an example of a cross-section view of a mould filled with wax.
Fig. 5 shows an example of ceramic pressing of a second layer of a dental restoration.
Fig. 6 shows an example of virtually designing a first layer and a second layer of a dental restoration.
Fig. 7 shows examples of moulds.
Fig. 8 shows a prior art dental restoration.
Fig. 9 shows an example of a restoration comprising a first layer, a second layer, and a third layer.
Fig. 10 shows an example of an investment mould for ceramic pressing of a restoration.
Fig. 11 shows an example of a mould for wax injection with a retention structure for retaining the first layer in the manufactured mould.
Fig. 12 shows examples of sprues.
Fig. 13 shows an example where sprues are designed for a full arch bridge.
Fig. 14 shows an example of how a retention pin of the mould can be virtually designed.
Fig. 15 shows an example of how the retention pin can be virtually designed.
Fig. 16 shows an embodiment in which an entry hole is defined.
Fig. 17 shows an embodiment for a denture.
Fig. 18 shows a schematic of a system according to an embodiment of the present invention.
Fig 19 shows a schematic of a user interface according to an embodiment of the invention.

### Detailed description

In the following description, reference is made to the accompanying figures, which show by way of illustration how the invention may be practiced.

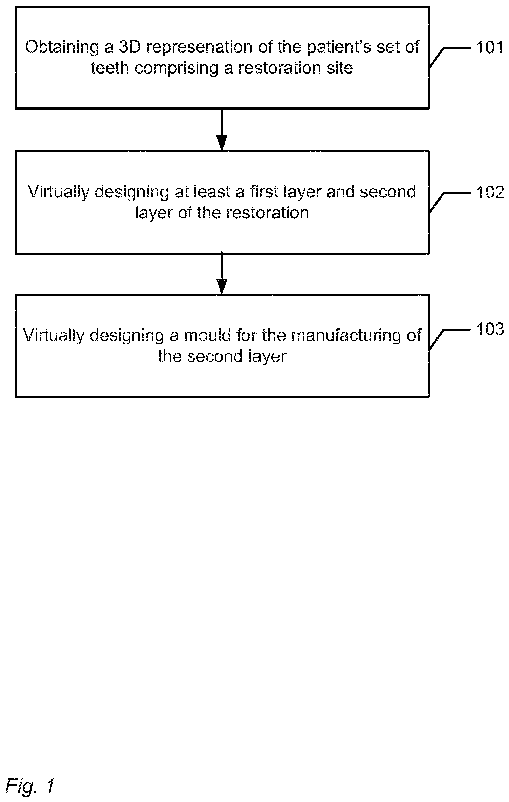

Fig. 1 shows an example of a flowchart of a method for designing a virtual mould used for manufacturing a corresponding physical mould adapted to be used in manufacturing of a dental restoration for a patient.

In step 101 a 3D representation of the patient's set of teeth is obtained, where the set of teeth comprises a restoration site where the manufactured dental restoration is adapted to be arranged.

In step 102 a first layer and a second layer of the dental restoration is virtually designed, where the first layer is adapted to match the restoration site and is part of a first portion of the dental restoration, and where the combination of the first portion and the second layer provides at least part of the dental restoration.

In step 103 the mould is virtually designed, where the mould is adapted for the manufacturing of the second layer, and where the inside surface of the mould is adapted to match the combination of the second layer and the first portion, and where the inside surface of the mould corresponds to the shape of the dental restoration.

Fig. 1 may also show an example of a flowchart of a method for virtually designing a mould adapted to be used for manufacturing a dental restoration for a patient, where the first layer is a frame layer and the second layer is an anatomy layer.

In step 101 a 3D representation of the patient's set of teeth is obtained, where the set of teeth comprises a restoration site where the manufactured dental restoration is adapted to be arranged.

In step 102 the first layer may be a frame layer and the second layer may be an anatomy layer of the dental restoration, and these layers are virtually designed, and the combination of the frame layer and the anatomy layer provides at least part of the dental restoration.

In step 103 a mould is virtually designed, where the inside surface of the mould is designed to match the combination of the anatomy layer and the frame layer, whereby the inside surface of the mould corresponds to the shape of the dental restoration.

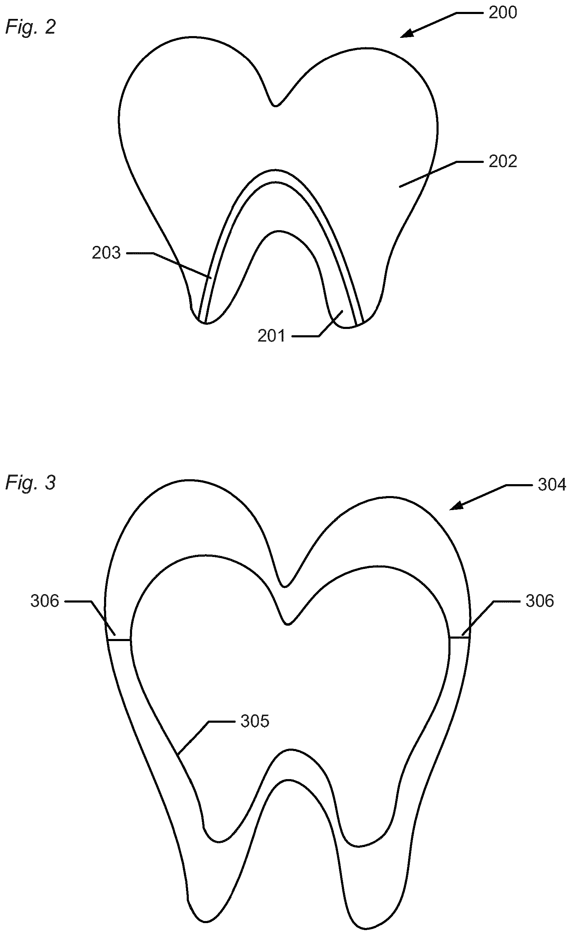

Fig. 2 shows an example of a cross-section view of a dental restoration 200 comprising a first layer 201, which can be a frame layer, a second layer 202, which can be an anatomy layer forming the outermost surface of the dental restoration, and an opaque coating 203 or liner coating.

The first layer 201 can be the frame of the dental restoration, and the first layer is adapted to fit on the restoration site of the patient's set of teeth.

The second layer 202 can be the anatomy layer, and is the crown of the restoration, which is visible in the patient's mouth, when the restoration is mounted.

The first layer may be made of metal, and for avoiding that any metal frame layer can be seen through the partly translucent second layer, e.g. anatomy layer, an opaque coating 203 or liner coating may be applied on the first layer. The opaque coating is typically white, such that the restoration resembles the natural colour and look of a real tooth.

Fig. 3 shows an example of a cross section view of a mould 304 for forming a dental restoration.

The inside surface 305 of the mould 304 is designed to match the combination of the second layer, e.g. anatomy layer, and the first layer, e.g. frame layer as seen in fig. 2, whereby the inside surface 305 of the mould 304 corresponds to the shape of the dental restoration as seen in fig. 2.

The mould comprises join lines 306 for assembling and disassembling the mould.

The mould is shown to be a mould for wax injection, but it is understood that the mould can also be an investment mould for ceramic pressing of the restoration.

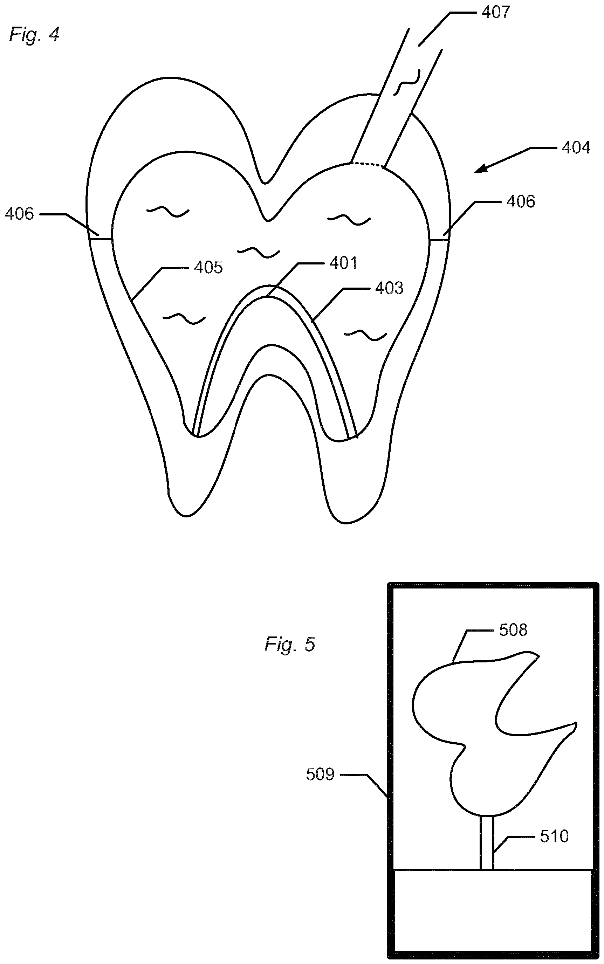

Fig. 4 shows an example of a cross-section view of a mould filled with wax.

The mould 404 has first been disassembled by splitting the mould in at least two pieces by the join lines 406.

Then the first portion comprising the first layer 401, e.g. frame layer, with an opaque coating 403 applied to it has been arranged in the mould in its correct location, which is where the inside surface 405 of the mould 404 matches the jaw facing surface of the first layer 401 of the first portion.

Then the mould 404 has been assembled at the join lines 406.

Then wax, indicated by the wavy lines, is filled or poured into the mould 404, such that the wax fills up the entire space of the mould 404 along the inside surface of the mould 405 and along the opaque coating 403 on the first layer 401. The wax is filled into the mould 404 by means of a wax inlet or sprue 407.

Afterwards the wax is allowed to harden in the mould, whereby the wax will acquire the shape of the second layer, e.g. anatomy layer, of the restoration. Finally, the mould is disassembled by the join lines 406, and the hardened wax with the shape of the second layer of the restoration can be removed from the mould.

If the restoration should comprise more layers, such as a third layer, a fourth layer etc., then this third layer, fourth layer etc. may be made on or attached on the first layer to define the first portion of the dental restoration, before the first portion is arranged in the mould for manufacturing the wax of the second layer. The first layer, the third layer and the fourth layer etc. may also have been made by means of a mould.

Fig. 5 shows an example of ceramic pressing of a second layer, e.g. an anatomy layer, of a dental restoration.

The hardened wax 508 with the shape of the second layer, e.g. the anatomy layer, of the dental restoration is placed in a container 509 suitable for resisting high pressure and high temperature. The container may comprise an inlet for gypsum, an outlet for wax, and an inlet for ceramics (not shown).

Gypsum is poured into the container, whereby the gypsum will encapsulate the hardened wax 508.

After the gypsum has hardened, the wax is melted out of the container.

Ceramics is then pressed into the container, whereby the ceramics will fill out the space left by the wax in the gypsum, and the ceramics will thus acquire the shape of the lost wax, which had the shape of the designed second layer, e.g. anatomy layer, of the restoration, whereby the ceramic second layer has been created.

The container is opened and the gypsum around the ceramic second layer is removed.

The ceramic second layer may be fired in an oven for obtaining robustness and strength.

Furthermore, sprues 510 on the ceramics second layer may be manufactured during the ceramic pressing process in the container, and these sprues may be cut of, e.g. after firing, colouring, staining etc. of the second layer.

If the restoration should comprise more layers, such as a third layer, a fourth layer etc., then this third layer, fourth layer etc. may be made on or attached on the first layer forming the first portion, before this is arranged in the mould for manufacturing the wax of the second layer.

Instead of placing wax in the container and performing the ceramic overpressing, the container can be an investment mould, where the ceramic restoration can be directly manufactured by virtually designing the cavity of the investment mould to match the restoration. Then the steps of handling the wax and the gypsum can be avoided.

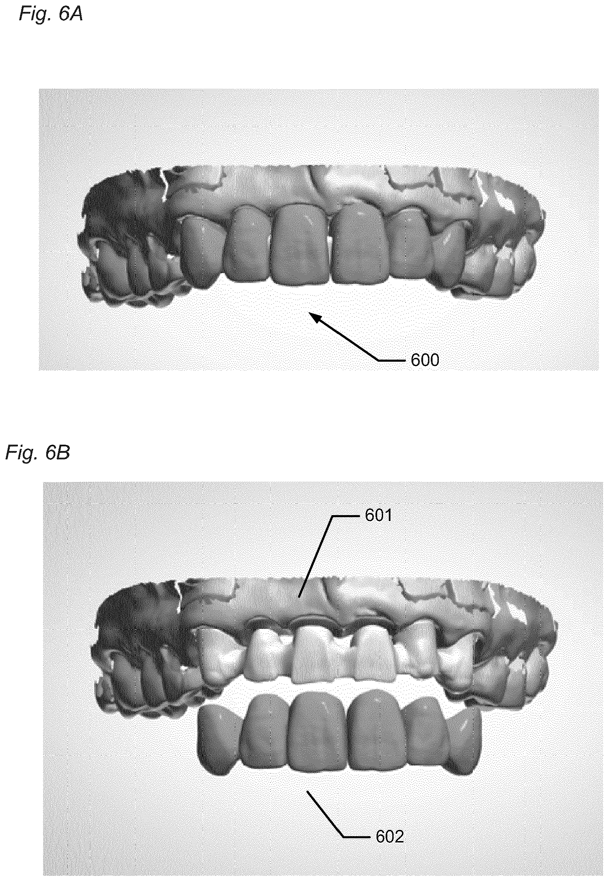

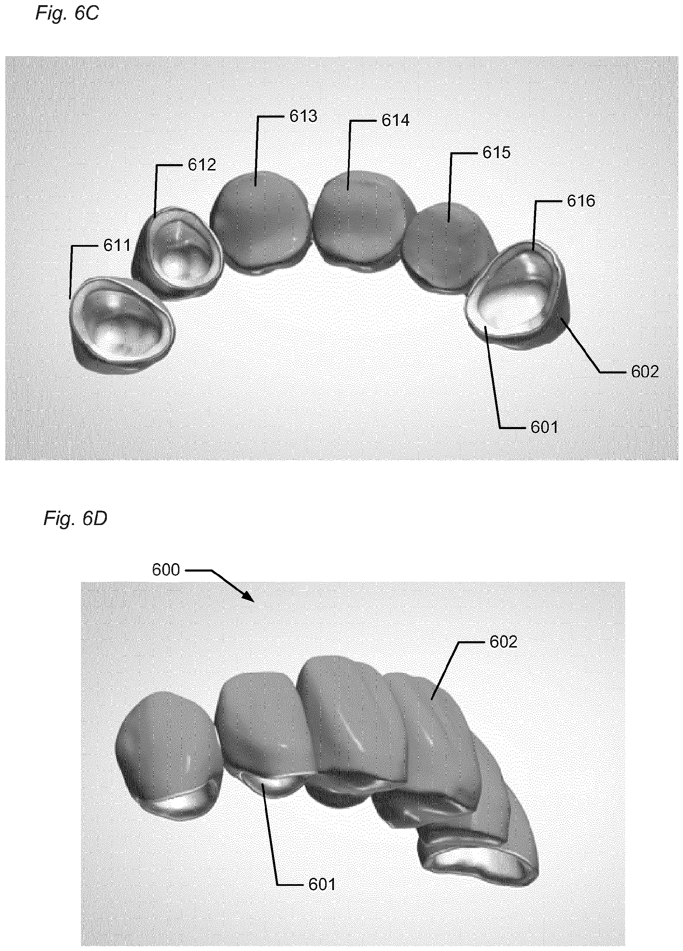

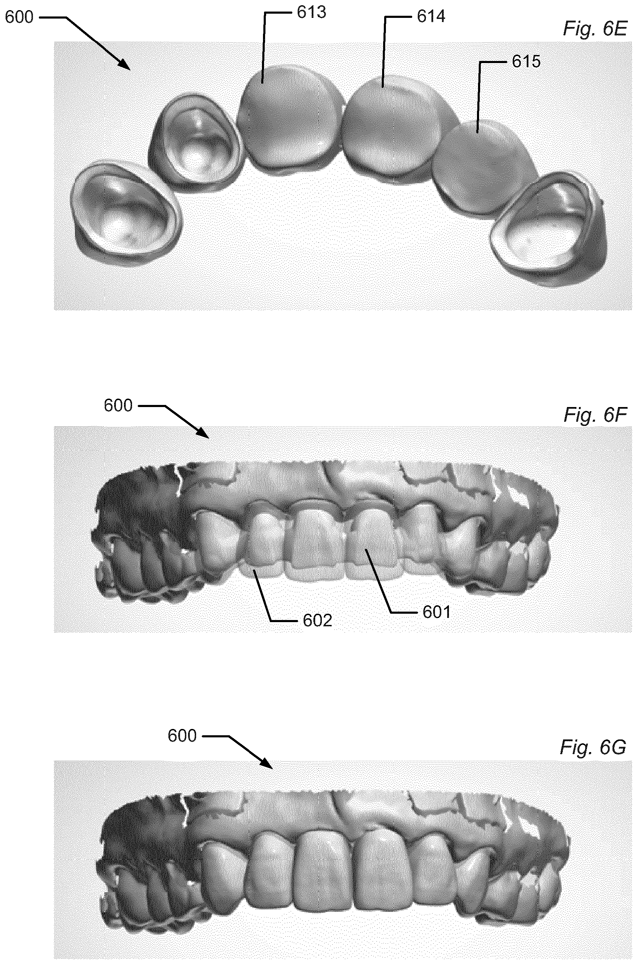

Fig. 6 shows an example of virtually designing a first layer, e.g. a frame layer, and a second layer, e.g. an anatomy layer, of a dental restoration.

Fig. 6A) shows a digital design of a dental restoration 600 in the form of a bridge comprising crowns and pontics. The crowns are adapted to fit on restorations sites in the form of preparations in the patient's mouth, and the pontics are adapted to fit on restoration sites in the form of gingival in the patient's mouth.

Fig. 6B) shows an example where the first layer 601, e.g. frame layer, and the second layer 602, e.g. anatomy layer, have been separated for visualization.

Fig. 6C) shows an example where the digital design of the dental restoration 600 is seen from the jaw facing surface. The first layer 601 is seen innermost and the second layer 602 is seen outermost.

Parts 611, 612 and 616 are crowns for preparations, and parts 613, 614 and 615 are pontics for resting over/on gingival.

Fig. 6D) shows an example where the digital design of the dental restoration 600 comprising the first layer 601, e.g. frame layer, and the second layer 602, e.g. anatomy layer, is seen partly from the side and partly from below.

Fig. 6E) shows an example of how the digital design of the dental restoration 600 can look when the entire surfaces of the dental restoration, for example including the jaw facing surface of the pontics 613, 614 and 615, are designed. By means of the present method for manufacturing the dental restoration, this design can be manufactured as shown without a dental technician having to finish the dental restoration manually at the margin line, which is the conventional process.

Fig. 6F) shows an example of the digital design of the dental restoration 600 seen from the front where the second layer 602, e.g. anatomy layer, is made transparent such that the first layer 601, e.g. frame layer, can be seen under the second layer.

Fig. 6G) shows an example of the digital design of the dental restoration 600 seen from the front.

If the restoration should comprise more layers, such as a third layer, a fourth layer etc., then this third layer, fourth layer etc. may be digitally designed before or after the second layer and/or the first layer..

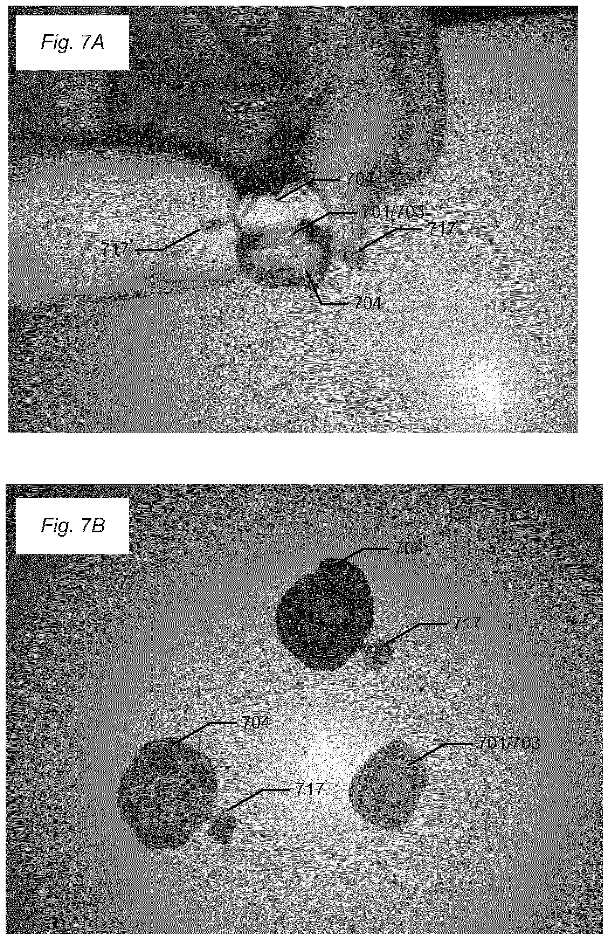

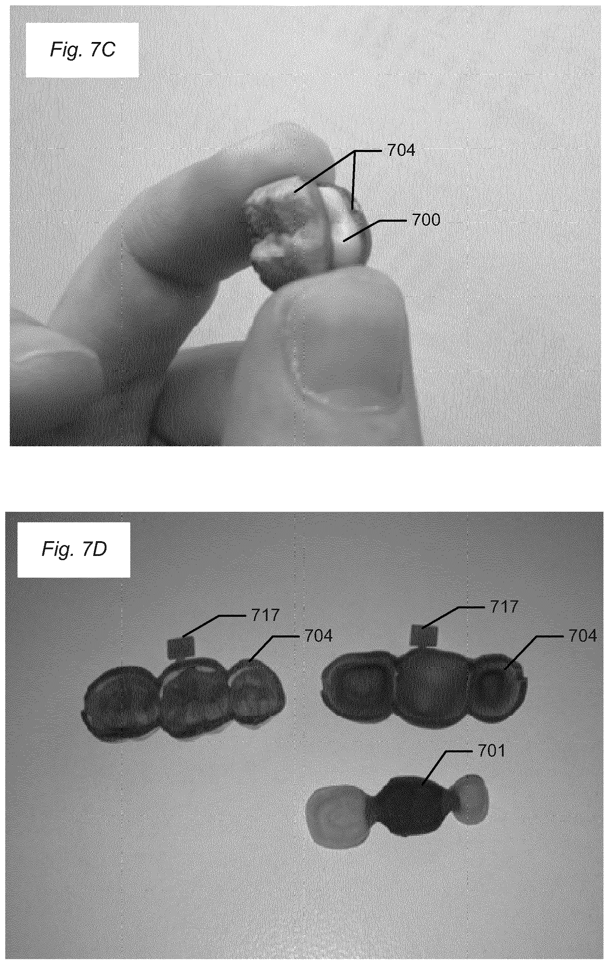

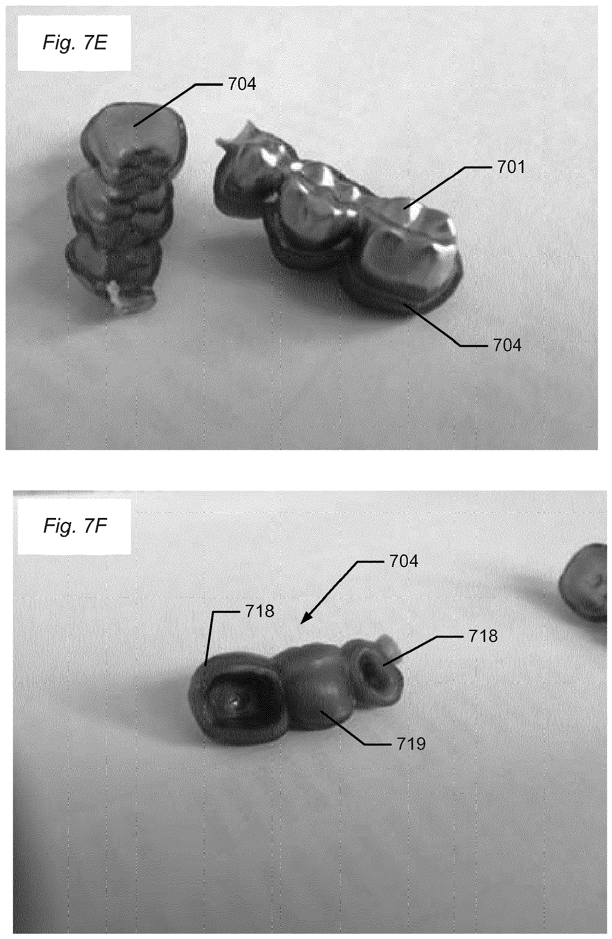

Fig. 7 shows examples of moulds.

Fig. 7A) shows a photo of a partly open mould 704 for a single crown or for wax to be used for manufacturing a single crown. Inside the mould 704 a first layer 701, e.g. a frame layer, for a dental restoration is placed. An opaque coating 703 may have been applied to the first layer 701. Wax may be applied in the mould on the first layer, whereby the crown can be manufactured.

The mould 704 comprises two parts. Closing pins 717 are arranged on the mould for easy opening and closing of the mould. One part of the closing pin 717 is arranged on one part of the mould, and the other part of the closing pin 717 is arranged in the other part of the mould.

Fig. 7B) shows an example of a disassembled mould 704 for a single crown.

Next to the two mould parts 704, the first portion consisting of the first layer 701, e.g. frame layer, with the opaque coating 703 is arranged. The closing pins 717 on the mould are also seen.

Fig. 7C) shows an example of a partly open mould 704 with the final restoration 700 inside to show that the final restoration matches the mould exactly. Thus the second layer, e.g. anatomy layer, has been added to the first layer, e.g. frame layer, and opaque coating.

Fig. 7D) shows an example of a disassembled mould 704 for a three unit bridge. Below the two mould parts 704, the first layer 701, e.g. frame layer, is arranged. Closing pins 717 are arranged on the mould for easy opening and closing of the mould. One part of the closing pin 717 is arranged on one part of the mould, and the other part of the closing pin 717 is arranged in the other part of the mould.

Fig. 7E) shows an example of a disassembled mould 704 for a three unit bridge with the first layer 701, here a frame layer, arranged in one of the two mould parts 704.

Fig. 7F) shows an example of a mould 704 for a three unit bridge. The left and right units 718 of the bridge are for crowns to be mounted on preparations in the patient's mouth. The unit 719 in the middle is for a pontic to rest at the gingival in the patient's mouth.

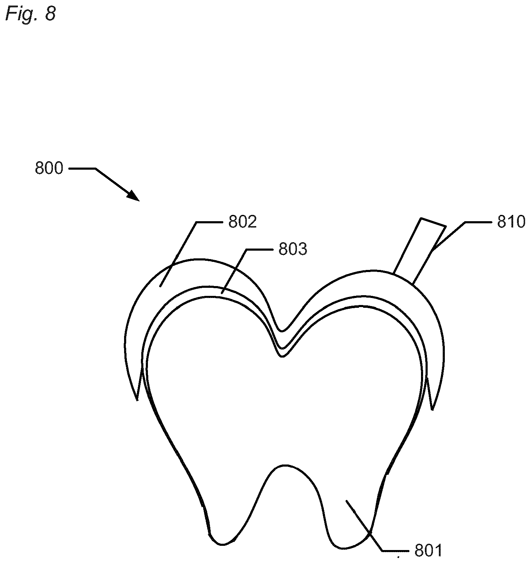

Fig. 8 shows a prior art dental restoration.

The prior art dental restoration 800 comprises a first layer 801, e.g. a frame layer, a second layer 802, e.g. an anatomy layer, and an opaque coating 803 on the first layer. A sprue 810 is attached to the second layer due to the ceramic press process. Due to the manufacturing process of prior art restorations, the restoration cannot be finished automatically at undercuts and at the margin line, which can be seen by that the second layer only covers the first layer until the thickest point on the first layer, the equator line, thus a dental technician must manually finish the second layer down until the margin line before the restoration is mounted in the patient's mouth.

Due to the manufacturing by means of the mould according to the present method, the entire restoration can be finished automatically, whereby a dental technician should not manually finish the anatomy layer at the margin line.

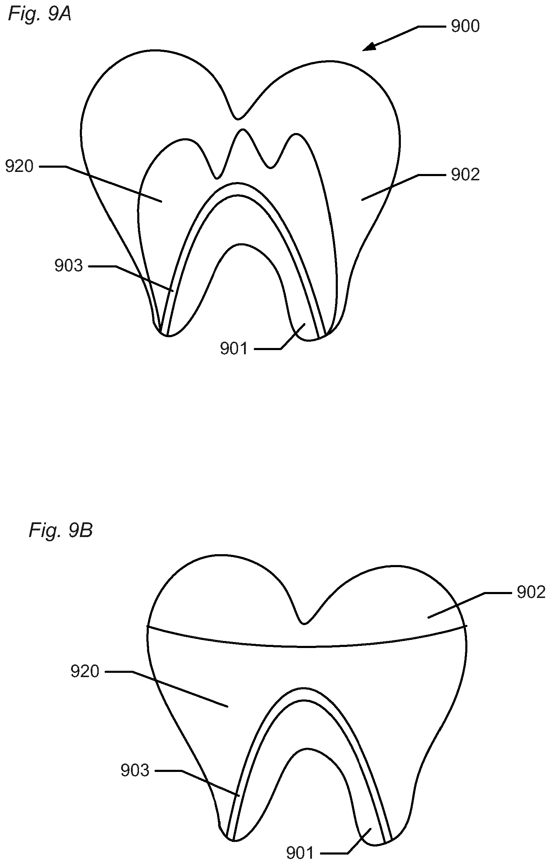

Fig. 9 shows examples of a restoration comprising a first layer, a second layer, and a third layer.

Fig. 9A) shows an example, where the restoration 900 is shown in a schematic cross-section view, and the dental restoration 900 comprises a first layer 901, a second layer 902, a third layer 920, and an opaque coating 903 or liner coating on the first layer 901. The second layer encapsulates the first portion defined by the opaque coating, the third layer and the first layer.

The first layer 901 can be the frame of the dental restoration, and the first layer is adapted to fit on the restoration site of the patient's set of teeth.

The second layer 902 can be the incisal layer, and is the outermost part of the restoration, which is visible in the patient's mouth, when the restoration is mounted.

The third layer 920 can be the dentin layer or mamelon layer, which is the layer between the frame layer and the incisal layer. An advantage of including this kind of third layer is that the visual appearance of the restoration can be made more lifelike when the third layer mimics the shape of the patient's natural dentin layer or mamelon layer.

The first layer may be made of metal, and for avoiding that any metal frame layer can be seen through the partly translucent second layer and third layer, an opaque coating 903 or liner coating may be applied on the first layer. The opaque coating is typically white, such that the restoration resembles the natural colour and look of a real tooth.

Fig. 9B) shows an example, where the restoration 900 is shown in a schematic cross-section view, and the dental restoration 900 comprises a first layer 901, a second layer 902, a third layer 920, and an opaque coating 903 or liner coating on the first layer 901. The third layer encapsulates the first layer, and the second layer is a layer on top of the third layer.

The first layer 901 can be the frame of the dental restoration, and the first layer is adapted to fit on the restoration site of the patient's set of teeth.

The second layer 902 is the outermost part of the restoration, which is visible in the patient's mouth, when the restoration is mounted.

The third layer 920 is the layer between the first layer and the second layer.

The first layer may be made of metal, and for avoiding that any metal frame layer can be seen through the partly translucent second layer and third layer, an opaque coating 903 or liner coating may be applied on the first layer. The opaque coating is typically white, such that the restoration resembles the natural colour and look of a real tooth.

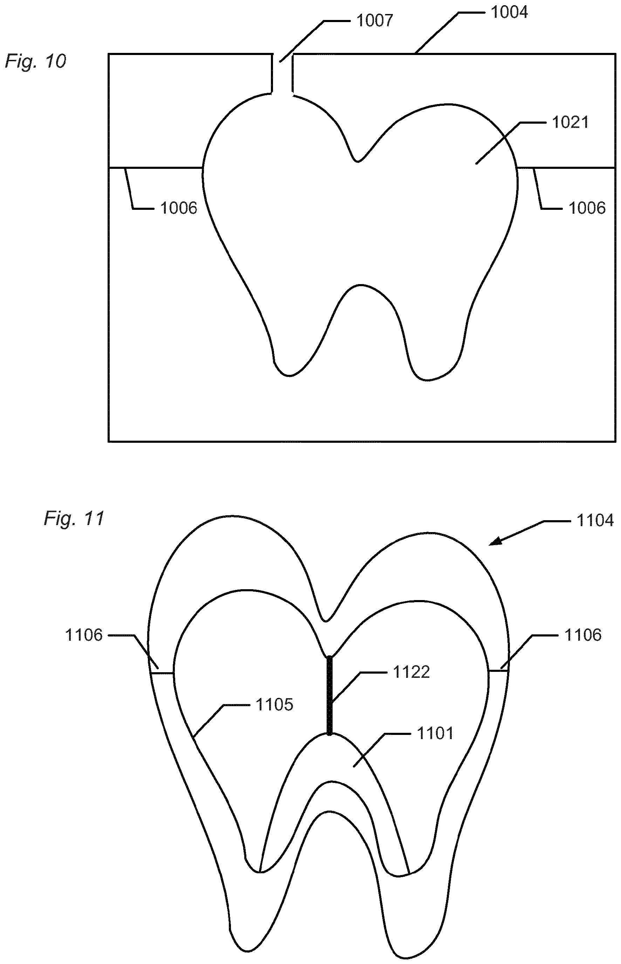

Fig. 10 shows an example of an investment mould for ceramic pressing of a restoration.

The investment mould 1004 is a solid block with a cavity 1021 inside shaped with the form of the restoration to be made. In inlet 1007 is provided in the investment mould 1004 for pressing in the ceramics for the second layer of the restoration. The first portion made of the first layer and any third layer, fourth layer etc. may be arranged inside the cavity 1021 (not shown) such that the second layer can be made on top of the other layers.

The investment mould 1004 comprises join lines 1006 such that the investment mould can be dissembled both for arranging the other layers in the mould before the ceramic pressing of the second layer and for removing the final restoration from the mould after the ceramic pressing.

The investment mould can be made of an investment material, such as gypsum.

Fig. 11 shows an example of a mould for wax injection with a retention structure applicable for retaining the first portion of the dental restoration in the manufactured mould. In this example, the first portion consists of the first layer.

The inside surface 1105 of the mould 1104 is designed to match the combination of at least the second layer (not shown) and the first layer 1101. The mould comprises join lines 1106 for assembling and disassembling the mould.

The mould 1104 comprises a retention structure 1122 for retaining the first layer 1101 in the manufactured mould, when for example wax is poured into the mould. If the first layer is displaced or moved when the wax is applied in the mould, then the first layer will not be arranged correctly relative to the wax layer that is being made, where the wax layer can be the second layer if there are only these two layers, or where the wax layer can be a third layer, if there are more than two layers.

The retention structure 1122 is in the form of a retention pin, which is fixed to the upper part of the mould 1104, and which touches the first layer 1101, when the mould is assembled and the first layer is arranged in the mould. Since the retention pin is made of wax, the retention pin will just mix into the wax applied to the mould for creating the wax copy of the next layer.

The mould is shown to be a mould for wax injection, but it is understood that the mould can also be an investment mould for ceramic pressing of the restoration or a mould for manufacturing the second layer in an acrylic material. If the mould is an investment mould, then the retention structure may be made of a different material, since then ceramics and not wax will be applied in the mould. In this case the retention pin may be made of ceramics, such that the ceramic retention pin will mix into the ceramic applied in the mould for creating the next layer.

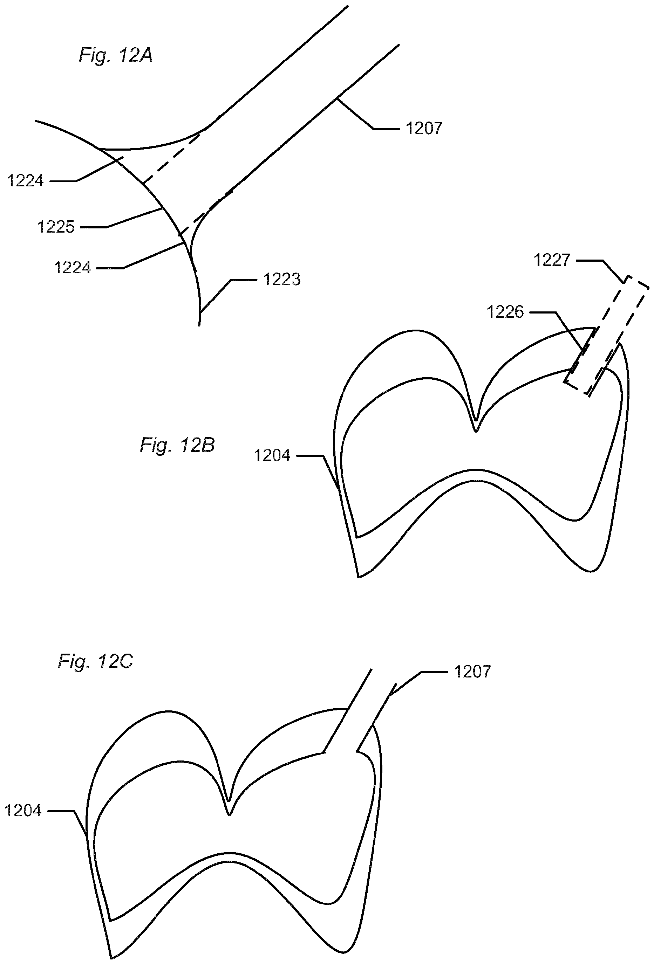

Fig. 12 shows examples of sprues.

Fig. 12a) shows an example of the traditional way of attaching a sprue 1207 on a wax copy 1223 of a restoration.

The sprue 1207 may be made of wax, and for attaching the sprue on the wax copy 1223 of the restoration, the end of the sprue is softened or melted by heating such that the two wax surfaces can melt together thereby fixing the sprue to the wax copy. But when the end of the sprue is melted it will typically not create an attachment to the wax copy of the restoration with straight angles, instead the end of the sprue will level off such that the transition between the sprue and the wax copy of the restoration spreads over a greater area 1224 than the cross section area 1225 of the sprue before melting, as seen in fig. 12a).

Fig. 12b) shows an example where a protrusion 1226 has been designed inside the mould 1204 for wax injection such that a hole will be formed in the wax copy of the restoration, when a pin 1227 is arranged in the protrusion 1226. Thus no sprue should be melted onto the wax copy of the restoration.

Fig. 12c) shows an example where a sprue 1207 has been designed on the outside surface of the mould 1204 for wax injection, whereby no sprue should be melted onto the surface of the wax copy.

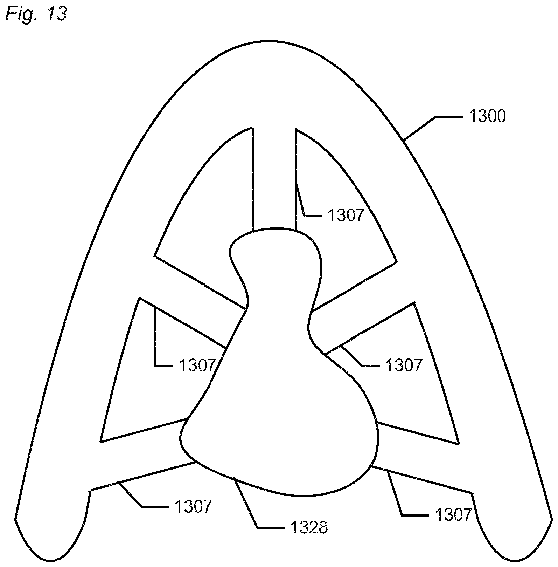

Fig. 13 shows an example where sprues are designed for a full arch bridge.

The restoration 1300 is a full arch bridge, and a number of sprues 1307 are designed for the restoration. The sprues 1307 are all connected to the common inlet/outlet 1328.

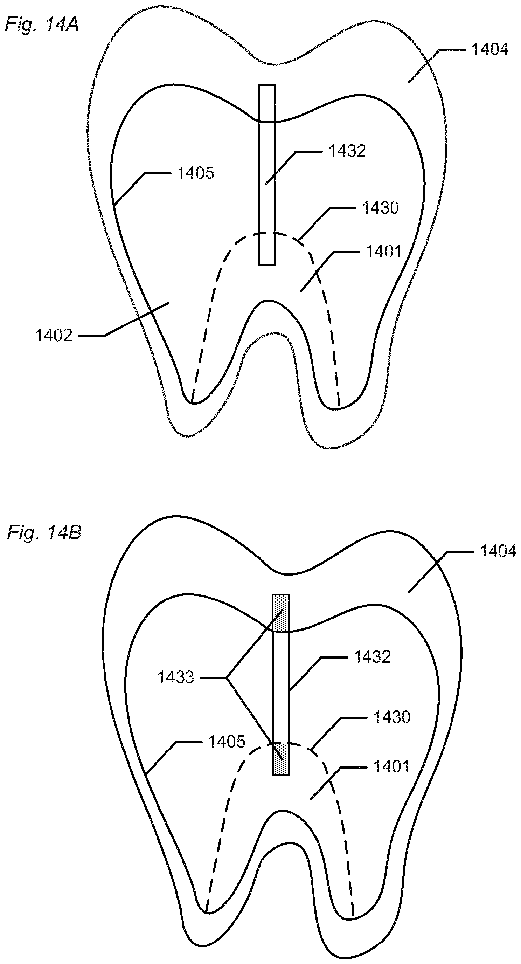

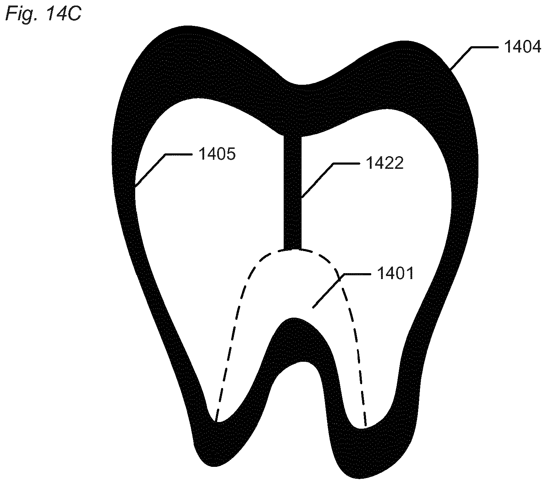

Fig. 14 shows an example of how a retention pin of the mould can be virtually designed.

In this example, the first portion of the dental restoration consists of the first layer, such that the virtual first portion consists of a virtual first layer 1401 having an outer surface 1430. The virtual first layer 1401 is arranged in the virtual mould 1404, such that the inner surface 1405 of the virtual mould together with the outer surface 1430 of the virtual first layer defines a space for the virtual second layer 1402.

In Fig. 14A a virtual pin structure 1432 is generated and arranged such that it intersects the inside surface 1405 of the virtual mould and the outer surface 1430 of the virtual first layer 1401.

In Fig. 14B the portions 1433 of the virtual pin structure 1432 extending outside the inner surface 1405 of the virtual mould and across the outer surface 1430 of the virtual first layer 1401 are indicated. These portions 1433 of the virtual pin structure are cut away using standard CAD software algorithms for subtracting the virtual mould 1404 and the virtual first layer 1401 from the virtual pin structure 1432 whereby the virtual retention pin is formed.

The virtual retention pin 1422 can then be virtually combined with the virtual structure defined by the inner and outer surfaces of the virtual mould 1404 to become an integrated part of the virtual mould. This can be done using standard CAD software algorithms for combining two objects into one. Subsequently, the physical mould can be manufactured based on the virtual mould.

Fig. 14C shows a cross section of the resulting mould 1404, where the retention pin 1422 extends from the inner surface 1405 of the mould towards the region where the first layer 1401 is to be placed. The retention pin 1422 can engage the first portion and is hence applicable in retaining the first portion of the dental restoration in its correct position in the mould during the manufacture of the second layer.

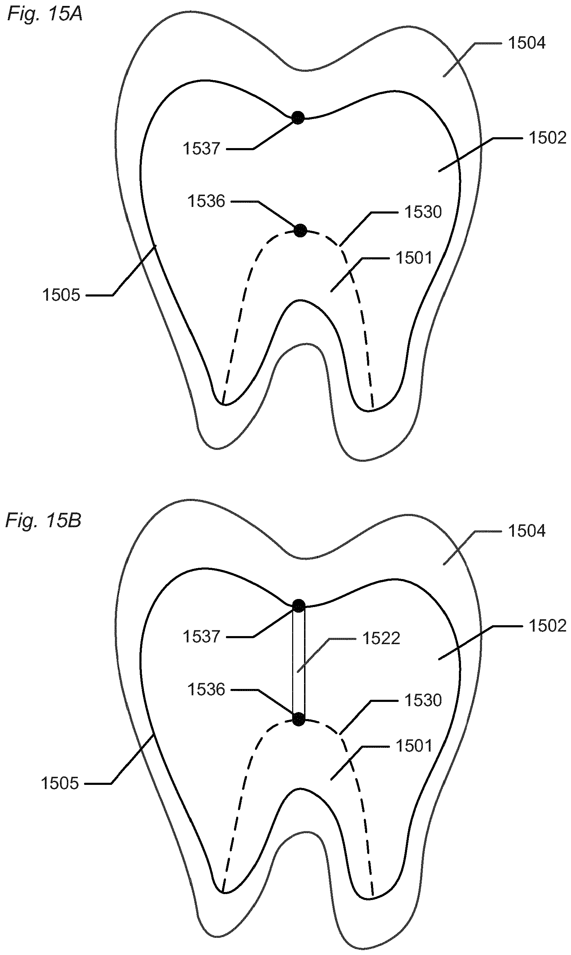

Fig. 15 shows an example of designing the virtual retention pin. In the illustrated Example, the first portion consists of the first layer.

Fig. 15A shows the virtual first layer 1501 arranged in the virtual mould 1504, such that the inner surface 1505 of the virtual mould together with the outer surface 1530 of the virtual first layer 1501 defines a space for the virtual second layer 1502. A first point 1536 is defined at the outer surface 1530 of the virtual first layer 1501 and a second point 1537 is defined at the inner surface 1505 of the virtual mould 1504. The second point is used to mark from which part of the mould inner surface 1505 the virtual retention pin will extend.

The second point 1537 may also be defined by indicating a point on the corresponding surface of the virtually designed second layer 1502 since this will coincide with the inner surface 1505 of the virtual mould 1504 over a large part of the inner mould surface.

The virtual retention pin 1522 is then generated by forming a virtual structure which extends between the second point 1537 and the first point 1536 on the opposing surface of the virtual first layer as illustrated in Fig. 15B.

The virtual retention pin 1522 is virtually combined with the virtual structure defined by the inner and outer surfaces of the virtual mould 1504 to become an integrated part of the virtual mould. This can be provided using standard CAD software algorithms. A physical mould can then be manufactured based on the virtual mould with a cross section equivalent to that illustrated in Fig. 14C.

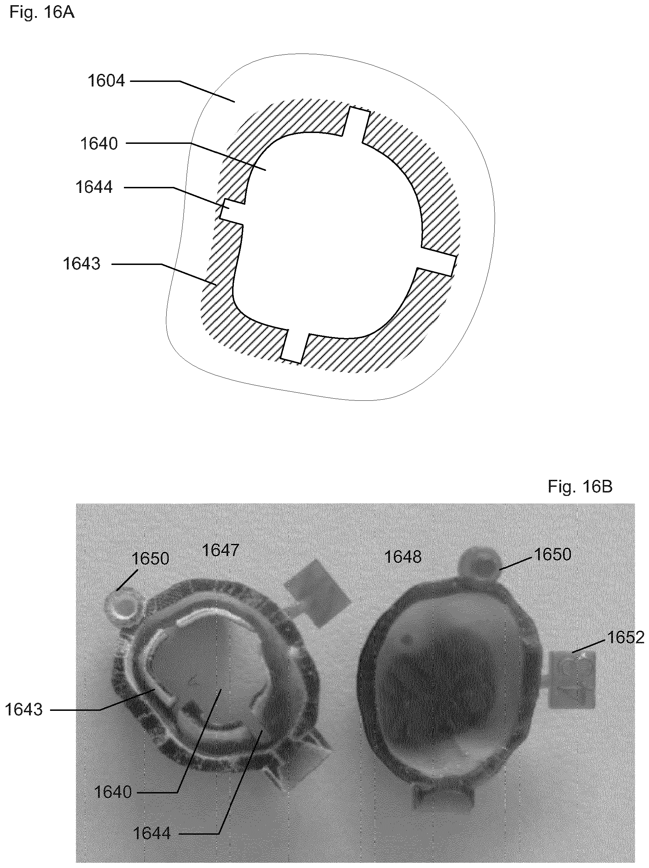

Fig. 16 shows an embodiment in which an entry hole is defined.

Fig. 16A shows a schematic cross section of the virtual mould 1604 as seen from the center of the mould towards the position of the first portion of the dental restoration in the mould. The entry hole 1640 is defined in the virtual mould 1604 e.g. by creating a 3D spline on the inner or outer surface of the virtual mould or by creating a virtual cylindrical structure and virtually removing the part of the mould located within this 3D spline or within the virtual cylindrical structure.

The cross sectional area of the entry hole is preferably in the range of about 50 % to about 90 % of the cross sectional area of the first portion of the dental restoration, such that when the mould is manufactured in a flexible material the entry hole provides that the first portion of the dental restoration can be pushed into the manufactured mould through said entry hole. Inside the mould, the first portion is supported by the mould material at the edge of the entry hole indicated by the area 1643 in Fig. 16A.

In order to increase the easy of pushing the first portion in the manufactured mould, indentations 1644 can be virtually defined in the perimeter of the entry hole 1640.

Fig. 16B shows images of a manufactured mould having a first part 1647 and a second part 1648 which can be assembled and held together by an assembly structure 1650. The mould also has an ID tag 1652 for easy identification.

The entry hole 1640 is defined in the first part with indentations 1644 at the perimeter of the entry hole.

When the first portion of the dental restoration is arranged in the mould it rests on the area 1643 surrounding the entry hole.

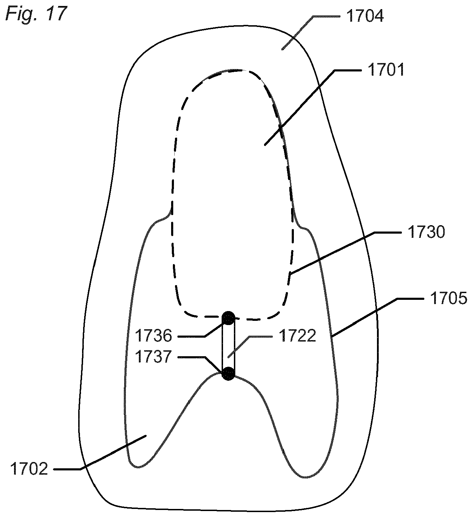

Fig. 17 shows an embodiment for manufacturing a mould for a denture.

When virtually designing a mould 1704 for the manufacture of a denture the first layer can be the denture teeth 1701 and the restoration site can be the alveolar ridge of the patient. The second layer 1702 is then the gingiva part of the denture and is designed to match the gingiva facing portion of the denture teeth and the restoration site. In cases where pre-manufactured denture teeth are used, the first layer may have been virtually designed by the manufacturer of the denture teeth, such that the virtually designing the first layer and the second layer of the dental restoration comprises importing the virtually designed denture teeth provided by the manufacturer.

In the figure, the virtual first layer (denture teeth) 1701 arranged in the virtual mould 1704, such that the inner surface 1705 of the virtual mould together with the gingiva facing surface 1730 of the virtual first layer 1701 defines a space for the virtual second layer 1702.

When virtually designing a retention pin for this mould, a first point 1736 can then be defined at the gingiva facing surface 1730 of the virtual first layer 1701 and a second point 1737 at the inner surface 1705 of the virtual mould 1704. The second point is used to mark from which part of the mould inner surface 1705 the virtual retention pin will extend. The virtual retention pin 1722 is then generated by forming a virtual structure which extends between the second point 1737 and the first point 1736 on the opposing surface of the virtual first layer. The virtual retention pin 1722 is then virtually combined with the virtual structure defined by the inner and outer surfaces of the virtual mould 1704 to become an integrated part of the virtual mould. This can be provided using standard CAD software algorithms.

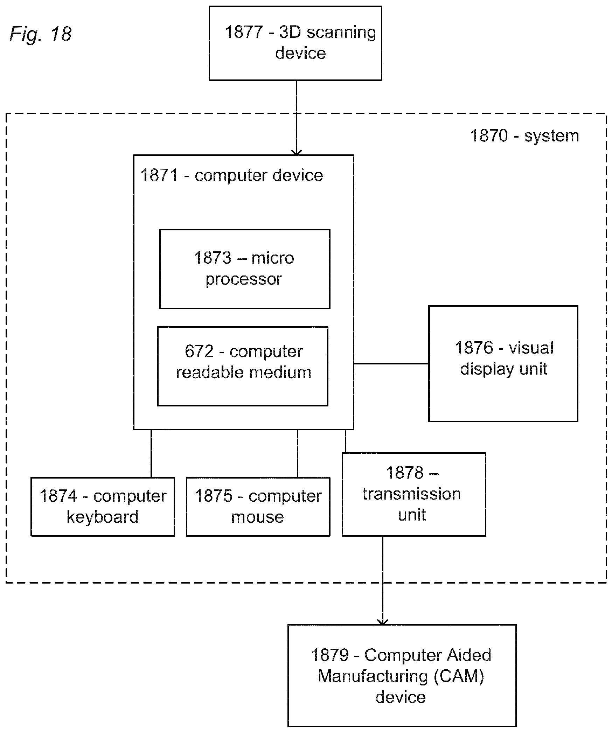

Fig. 18 shows a schematic of a system according to an embodiment of the present invention.

The system 1870 comprises a computer device 1871 comprising a computer readable medium 1872 and a microprocessor 1873. The system further comprises a visual display unit 1876, a computer keyboard 1874 and a computer mouse 1875 for entering data and activating virtual buttons visualized on the visual display unit 1876. The visual display unit 1876 can be a computer screen.

The computer device 1871 is capable of obtaining a 3D representation of the patient's set of teeth comprising a restoration site. The obtained 3D digital representations can be stored in the computer readable medium 1872 and provided to the processor 1873. The system 1870 is configured for allowing an operator to virtually design at least a first layer and a second layer of the dental restoration and to virtually design the mould, which is adapted for the manufacturing of the second layer. The first layer is part of a first portion of the dental restoration, and where the combination of the first portion and the second layer provides at least part of the dental restoration and the designed mould is adapted for the manufacturing of the second layer, where the inside surface of the mould is adapted to match the combination of the first portion and the second layer, where the inside surface of the mould corresponds to the shape of the dental restoration. The system is preferably capable of designing a virtual retention structure of the mould, where the corresponding physical retention structure is applicable in retaining the first portion of the dental restoration in its correct position in the mould. The virtually designed first portion and second layer can be displayed in a user interface depicted on the visual display unit 1876 allowing the operator to inspect the quality of the virtually designed mould and optionally to design the virtual retention structure, by e.g. defining its end points or by arranging a virtual pin structure in relation to the first portion and the virtual mould inner surface. This can be done using e.g. the computer mouse 1875 or the computer keyboard 1874. The computer device 1871 is configured for virtually designing the mould from the surfaces of the second layer and the first portion at least partly using the microprocessor 1873.

The computer device 1871 is further capable of receiving a digital 3D representation of the patient's set of teeth from a 3D scanning device 1877, such as the TRIOS intra-oral scanner manufactured by 3shape A/S, or capable of receiving scan data from such a 3D scanning device and forming a digital 3D representation of the patient's set of teeth based on such scan data. The received or formed digital 3D representation can be stored in the computer readable medium 1872 and provided to the microprocessor 1873.

The system comprises a unit 1878 for transmitting the virtual 3D model to e.g. a computer aided manufacturing (CAM) device 1879 for manufacturing the dental restoration or to another computer system e.g. located at a milling center where the dental restoration is manufactured. The unit for transmitting the virtual 3D model can be a wired or a wireless connection.

The 3D scanning of the patient's set of teeth using the 3D scanning device 1877 can be performed at a dentist while the designing of the dental restoration is performed at a dental laboratory. In such cases the digital 3D representation of the patient's set of teeth can be provided via an internet connection between the dentist and the dental laboratory.

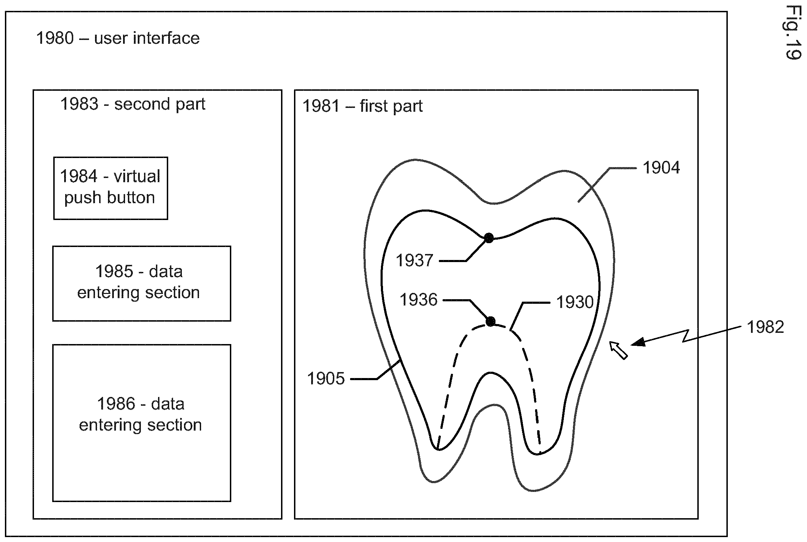

Fig 19 shows a schematic of a user interface according to an embodiment of the invention.

In Fig. 19 a first part 1981 of the user interface 1980 is seen in which an image of the virtually designed first and second layer, and mould is illustrated. The position of first 1936 and second 1937 points for creating a virtual retention pin can be adjusted using a virtual movement tool 1982. The second part 1983 of the user interface comprises a data entering sections 1985, 1986 for entering data relating to e.g. the thickness and shape of the retention pin. A virtual push button 1984 is configured for creating the virtual retention pin based on the positions of the first and second points 1936, 1937 taking into account the data entered in the data entering section 1986.

The user interface can be visualized on a visual display unit, such as a computer screen being part of a system configured for implementing the method according to the present invention.

Although some embodiments have been described and shown in detail, the invention is not restricted to them, but may also be embodied in other ways within the scope of the subject matter defined in the following claims.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

The features of the method described above and in the following may be implemented in software and carried out on a data processing system or other processing means caused by the execution of computer-executable instructions. The instructions may be program code means loaded in a memory, such as a RAM, from a storage medium or from another computer via a computer network. Alternatively, the described features may be implemented by hardwired circuitry instead of software or in combination with software.

## Claims

1. A method for designing a virtual mould used for manufacturing a corresponding physical mould adapted to be used in manufacturing of a dental restoration for a patient, wherein the method comprises:
- obtaining a 3D representation of the patient's set of teeth comprising a restoration site (101) where the manufactured dental restoration is adapted to be arranged;
- virtually designing at least a first layer and a second layer of the dental 2. restoration (102), where the first layer is part of a first portion of the dental restoration, and where the combination of the first portion and the second layer provides at least part of the dental restoration; and
- virtually designing the mould (103), which is adapted for the manufacturing of the second layer, where the inside surface of the mould is adapted to match the combination of the first portion and the second layer, where the inside surface of the mould corresponds to the shape of the dental restoration; where virtually designing the mould comprises designing a virtual retention structure (510) of the mould, where the corresponding physical retention structure is applicable in retaining the first portion of the dental restoration 2. (508) in its correct position in the mould.

2. The method according to claim 1, wherein the virtual retention structure comprises a virtual retention pin configured to provide that the corresponding physical retention pin of the manufactured mould extends from a point on the inside surface of the mould to a point on the opposing surface of the first portion of the dental restoration.

3. The method according to claim 2, wherein the designing of the virtual retention pin is configured to provide that it is an integrated part of the virtual mould.

4. The method according to any one or more of claims 2 or 3, where the virtual designing comprises defining a point on the inside surface of the virtual mould from which the virtual retention pin extends and a point on the opposing surface of a virtual first portion corresponding to the first portion of the dental restoration, and virtually generating the retention pin by forming a structure which extends between these two points.

5. The method according to any one or more of claims 2 to 4, where virtual designing the mould comprises:
- generating a virtual pin structure which intersects the inside surface of the virtual mould and the virtual first portion corresponding to the first portion of the dental restoration; and
- forming the virtual retention pin by subtracting the virtual first portion and the virtual mould from the virtual pin structure.

6. The method according to any one or more of the preceding claims, wherein the virtual retention structure comprises a virtual vacuum suction hole arranged at the part of the virtual mould where the first layer is to be arranged in the physical mould.

7. The method according to any one or more of the preceding claims, wherein the method comprises defining an entry hole in the virtual mould at the position of the first portion of the dental restoration, where the cross sectional area of the entry hole is in the range of about 50 % to about 90 % of the cross sectional area of the first portion of the dental restoration.

8. The method according to claim 7, wherein the method comprises virtually designing indentations in the perimeter of the entry hole.

9. The method according to any one or more of the preceding claims, wherein the first layer is a frame layer or a coping and the second layer is an anatomy layer, an incisal layer or an occlusal layer of the dental restoration.

10. The method according to any one or more of the preceding claims, wherein the method comprises virtually designing a third layer of the dental restoration, and where the third layer is comprised in the first portion of the dental restoration.

11. The method according to claim 10, wherein the first or the third layer is a dentin or mamelon layer.

12. The method according to any one or more of claims 1 to 8, wherein the dental restoration is a denture, the first portion the denture teeth and the second layer the gingiva part of the denture.

13. The method according to any one or more of the preceding claims, wherein the method comprises virtually designing a protrusion inside the mould.

14. The method according to any one or more of the preceding claims, wherein the method comprises virtually designing an entry hole in the mould.

15. A method for use in manufacturing a dental restoration (200) for a restoration
site of a patient, said dental restoration comprising a first layer and a second layer, the method comprising:
- designing a virtual mould using the method according to any one of claims 1 to 14;
- obtaining a first portion of the dental restoration, said first portion comprising the first layer (201),
- manufacturing a physical mould (304) from the designed virtual mould, where the physical mould is adapted to be used in manufacturing of the second layer (202) the dental restoration and comprises a retention structure (510) applicable for retaining the first portion of the dental restoration in its correct position in the mould;
- arranging the first portion (201) of the dental restoration inside the physical mould such that it is held in its correct position by use of said retention structure; and
- manufacturing the second layer (202) of said dental restoration using the manufactured physical mould with the first portion arranged inside.

## Patentansprüche

1. Verfahren zum Entwerfen einer virtuellen Form, die zum Herstellen einer entsprechenden physischen Form verwendet wird, die angepasst ist, um beim Herstellen einer Zahnrestauration verwendet zu werden, wobei das Verfahren umfasst:
Erhalten einer 3D-Darstellung des Satzes von Zähnen des Patienten, der die Restaurationsstelle (101) umfasst, wobei die hergestellte Zahnrestauration dazu angepasst ist, eingerichtet zu werden;
- virtuelles Entwerfen mindestens einer ersten Schicht und einer zweiten Schicht der Zahnrestauration (102), wobei die erste Schicht Teil eines ersten Abschnitts der Zahnrestauration ist, und wobei die Kombination des ersten Abschnitts und der zweiten Schicht mindestens einen Teil der Zahnrestauration bereitstellt; und
- virtuelles Entwerfen der Form (103), die für das Herstellen der zweiten Schicht angepasst ist, wobei die Innenoberfläche der Form dazu angepasst ist, zu der Kombination des ersten Abschnitts und der zweiten Schicht zu passen, wobei die Innenoberfläche der Form der Form der Zahnrestauration entspricht;
wobei das virtuelle Entwerfen der Form das Entwerfen einer virtuellen Haltestruktur (510) der Form umfasst, wobei die entsprechende physische Haltestruktur beim Halten des ersten Abschnitts der Zahnrestauration (508) in ihrer korrekten Position in der Form anwendbar ist.

2. Verfahren nach Anspruch 1, wobei die virtuelle Haltestruktur einen virtuellen Haltestift umfasst, der dazu konfiguriert ist vorzusehen, dass sich der entsprechende physische Haltestift der hergestellten Form von einem Punkt auf der Innenoberfläche der Form zu einem Punkt auf der entgegengesetzten Oberfläche des ersten Abschnitts der Zahnrestauration erstreckt.

3. Verfahren nach Anspruch 2, wobei das Entwerfen des virtuellen Haltestifts dazu konfiguriert ist vorzusehen, dass er ein integriertes Teil der ersten Form ist.

4. Verfahren nach einem oder mehreren der Ansprüche 2 oder 3, wobei das virtuelle Entwerfen das Definieren eines Punkts auf der Innenoberfläche der virtuellen Form umfasst, von dem ausgehend sich der virtuelle Haltestift erstreckt, und einen Punkt auf der entgegengesetzten Oberfläche eines virtuellen ersten Abschnitts, der dem ersten Abschnitt der Zahnrestauration entspricht, und virtuelles Erzeugen des Haltestifts durch Bilden einer Struktur, die sich zwischen diesen zwei Punkten erstreckt.

5. Verfahren nach einem der Ansprüche 2 bis 4, wobei das virtuelle Entwerfen der Form umfasst:
- Erzeugen einer virtuellen Stiftstruktur, die die Innenoberfläche der virtuellen Form und den virtuellen ersten Abschnitt, der dem ersten Abschnitt der Zahnrestauration entspricht, schneidet; und
- Bilden eines virtuellen Haltestifts durch Subtrahieren des virtuellen ersten Abschnitts und der virtuellen Form von der virtuellen Stiftstruktur.

6. Verfahren nach einem oder mehreren der vorstehenden Ansprüche, wobei die virtuelle Haltestruktur ein virtuelles Vakuumsaugloch umfasst, das an dem Teil der virtuellen Form, an dem die erste Schicht in der physischen Form eingerichtet werden soll, eingerichtet ist.

7. Verfahren nach einem oder mehreren der vorstehenden Ansprüche, wobei das Verfahren das Definieren eines Eintrittslochs in die virtuelle Form an der Position des ersten Abschnitts der Zahnrestauration umfasst, wobei die Querschnittfläche des Eintrittslochs in dem Bereich von etwa 50% bis etwa 90% der Querschnittfläche des ersten Abschnitts der Zahnrestauration liegt.

8. Verfahren nach Anspruch 7, wobei das Verfahren das virtuelle Entwerfen von Ausbuchtungen in dem Umfang des Eintrittslochs umfasst.

9. Verfahren nach einem oder mehreren der vorstehenden Ansprüche, wobei die erste Schicht eine Rahmenschicht oder eine Deckschicht ist, und die zweite Schicht eine Anatomieschicht, eine Schneidschicht oder eine Okklusionsschicht der Zahnrestauration ist.

10. Verfahren nach einem oder mehreren der vorstehenden Ansprüche, wobei das Verfahren das virtuelle Entwerfen einer dritten Schicht der Zahnrestauration umfasst, und wobei die dritte Schicht in dem ersten Abschnitt der Zahnrestauration enthalten ist.

11. Verfahren nach Anspruch 10, wobei die erste oder die dritte Schicht eine Zahnbein- oder Mamelonschicht ist.

12. Verfahren nach einem oder mehreren der Ansprüche 1 bis 8, wobei die Zahnrestauration ein Zahnersatz ist, wobei der erste Abschnitt der Zahnersatzzähne und die zweite Schicht der Zahnfleischteil des Zahnersatzes ist.

13. Verfahren nach einem oder mehreren der vorstehenden Ansprüche, wobei das Verfahren das virtuelle Entwerfen eines Vorsprungs innerhalb der Form umfasst.

14. Verfahren nach einem oder mehreren der vorstehenden Ansprüche, wobei das Verfahren das virtuelle Entwerfen eines Eintrittslochs in der Form umfasst.

15. Verfahren zur Verwendung beim Herstellen einer Zahnrestauration (200) für eine Restaurationsstelle eines Patienten, wobei die Zahnrestauration eine erste Schicht und eine zweite Schicht umfasst, wobei das Verfahren umfasst:
- Entwerfen einer virtuellen Form unter Verwenden des Verfahrens nach einem der Ansprüche 1 bis 14;
- Erhalten eines ersten Abschnitts der Zahnrestauration, wobei der erste Abschnitt die erste Schicht (201) umfasst,
- Herstellen einer physischen Form (304) aus der entworfenen virtuellen Form, wobei die physische Form dazu angepasst ist, beim Herstellen der zweiten Schicht (202) der Zahnrestauration verwendet zu werden und eine Haltestruktur (510) umfasst, die zum Halten des ersten Abschnitts der Zahnrestauration in seiner korrekten Position in der Form anwendbar ist;
- Einrichten des ersten Abschnitts (201) der Zahnrestauration innerhalb der physischen Form derart, dass sie durch Verwenden der Haltestruktur in ihrer korrekten Position gehalten wird;
und
- Herstellen der zweiten Schicht (202) der Zahnrestauration unter Verwenden der hergestellten physischen Form mit dem ersten Abschnitt darin eingerichtet.

## Revendications

1. Procédé de conception d'un moule virtuel utilisé pour la fabrication d'un moule physique correspondant adapté pour être utilisé dans la fabrication d'une restauration dentaire pour un patient, dans lequel le procédé comprend :
- l'obtention d'une représentation 3D de l'ensemble de dents du patient comprenant un site de restauration (101) où la restauration dentaire fabriquée est adaptée pour être agencée ;
- la conception virtuelle d'au moins une première couche et une deuxième couche de la restauration dentaire (102), où la première couche fait partie d'une première portion de la restauration dentaire, et où la combinaison de la première portion et de la deuxième couche fournit au moins une partie de la restauration dentaire ; et
- la conception virtuelle du moule (103), qui est adapté pour la fabrication de la deuxième couche, où la surface intérieure du moule est adaptée pour coïncider avec la combinaison de la première portion et de la deuxième couche, où la surface intérieure du moule correspond à la forme de la restauration dentaire ;
où la conception virtuelle du moule comprend la conception d'une structure de retenue virtuelle (510) du moule, où la structure de retenue physique correspondante est applicable dans la retenue de la première portion de la restauration dentaire (508) dans sa position correcte dans le moule.

2. Procédé selon la revendication 1, dans lequel la structure de retenue virtuelle comprend une broche de retenue virtuelle configurée pour prévoir que broche de retenue physique correspondante du moule fabriqué s'étend d'un point sur la surface intérieure du moule à un point sur la surface opposée de la première portion de la restauration dentaire.

3. Procédé selon la revendication 2, dans lequel la conception de la broche de retenue virtuelle est configurée pour prévoir qu'elle fait partie intégrante du moule virtuel.

4. Procédé selon une ou plusieurs quelconques des revendications 2 ou 3, où la conception virtuelle comprend la définition d'un point sur la surface intérieure du moule virtuel à partir duquel la broche de retenue virtuelle s'étend et un point sur la surface opposée d'une première portion virtuelle correspondant à la première portion de la restauration dentaire, et la génération virtuelle de la broche de retenue par formation d'une structure qui s'étend entre ces deux points.

5. Procédé selon une ou plusieurs quelconques des revendications 2 à 4, où la conception virtuelle du moule comprend :
- la génération d'une structure de broche virtuelle qui coupe la surface intérieure du moule virtuel et la première portion virtuelle correspondant à la première portion de la restauration dentaire ; et
- la formation de la broche de retenue virtuelle par soustraction de la première portion virtuelle et du moule virtuel de la structure de broche virtuelle.

6. Procédé selon une ou plusieurs quelconque des revendications précédentes, dans lequel la structure de retenue virtuelle comprend un trou d'aspiration de vide virtuel agencé au niveau de la partie du moule virtuel où la première couche doit être agencée dans le moule physique.

7. Procédé selon une ou plusieurs quelconques des revendications précédentes, dans lequel le procédé comprend la définition d'un trou d'entrée dans le moule virtuel au niveau de la position de la première portion de la restauration dentaire, où la surface de section transversale du trou d'entrée est dans la plage d'environ 50 % à environ 90 % de la surface de section transversale de la première portion de la restauration dentaire.

8. Procédé selon la revendication 7, dans lequel le procédé comprend la conception virtuelle d'indentations dans le périmètre du trou d'entrée.

9. Procédé selon une ou plusieurs quelconques des revendications précédentes, dans lequel la première couche est une couche d'armature ou une coiffe et la deuxième couche est une couche anatomique, une couche incisive ou une couche occlusale de la restauration dentaire.

10. Procédé selon une ou plusieurs quelconques des revendications précédentes, dans lequel le procédé comprend la conception virtuelle d'une troisième couche de la restauration dentaire, et où la troisième couche est comprise dans la première portion de la restauration dentaire.

11. Procédé selon la revendication 10, dans lequel la première ou la troisième couche est une couche de dentine ou de dentelure marginale.

12. Procédé selon une ou plusieurs quelconques des revendications 1 à 8, dans lequel la restauration dentaire est une prothèse dentaire, la première portion les dents de prothèse dentaire et la deuxième couche la partie gingivale de la prothèse dentaire.

13. Procédé selon une ou plusieurs quelconques des revendications précédentes, dans lequel le procédé comprend la conception virtuelle d'une saillie à l'intérieur du moule.

14. Procédé selon une ou plusieurs quelconques des revendications précédentes, dans lequel le procédé comprend la conception virtuelle d'un trou d'entrée dans le moule.

15. Procédé d'utilisation dans la fabrication d'une restauration dentaire (200) pour un site de
restauration d'un patient, ladite restauration dentaire comprenant une première couche et une deuxième couche, le procédé comprenant :
- la conception d'un moule virtuel en utilisant le procédé selon l'une quelconque des revendications 1 à 14 ;
- l'obtention d'une première portion de la restauration dentaire, ladite première portion comprenant la première couche (201),
- la fabrication d'un moule physique (304) à partir du moule virtuel conçu, où le moule physique est adapté pour être utilisé dans la fabrication de la deuxième couche (202) de la restauration dentaire et comprend une structure de retenue (510) applicable pour retenir la première portion de la restauration dentaire dans sa position correcte dans le moule ;
- l'agencement de la première portion (201) de la restauration dentaire à l'intérieur du moule physique de telle manière qu'elle est maintenue dans sa position correcte par utilisation de ladite structure de retenue ;
et
- la fabrication de la deuxième couche (202) de ladite restauration dentaire en utilisant le moule physique fabriqué avec la première portion agencée à l'intérieur.
